# EUROPEAN PATENT APPLICATION

(11) **EP 3 936 603 A1**
(43) Date of publication of application: **12.01.2022**
(21) Application number: 20778546.0
(22) Date of filing: 25.02.2020
(51) Int. Cl.: C12M 1/34

(54) **MEASUREMENT DEVICE, OPERATION PROGRAM FOR MEASUREMENT DEVICE, AND MEASUREMENT METHOD**

(30) Priority: 28.03.2019 JP 2019063660
(71) Applicant: FUJIFILM Corporation, Tokyo 106-8620 (JP)
(72) Inventor: OGURA, Takahiro, Ashigarakami-gun, Kanagawa 258-8538 (JP); ITO, Koju, Ashigarakami-gun, Kanagawa 258-8538 (JP)
(74) Representative: Hoffmann Eitle
(86) International application number: PCT/JP2020/007479
(87) International publication number: WO 2020/195458

(57) **Abstract**

Provided is a measurement device including a mounting unit in which a flow channel device is attachably and detachably mounted, the flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion; and a detection unit that optically distinguishes and detects a leakage state of each of a plurality of types of tracers having different sizes and leaking out from the first flow channel into the second flow channel through a defect occurring in the cell sheet in a case where an evaluation liquid is fed into the first flow channel, the evaluation liquid being used for evaluation of barrier properties of the cell sheet and containing the plurality of types of tracers in a mixed state.

## Description

### BACKGROUND

### Technical Field

The technique of the present disclosure relates to a measurement device, an operation program for a measurement device, and a measurement method.

### Background Art

There is known a flow channel device including a fine flow channel capable of feeding a liquid and a cell sheet in which a plurality of cultured cells are arranged in such a manner that the cells are bound to each other (see JP2018-522586A). Such a flow channel device is used, for example, to evaluate the barrier properties of a cell sheet.

An evaluation liquid containing a tracer is used to evaluate the barrier properties of the cell sheet. In a case where the cell sheet is defective, the tracer contained in the evaluation liquid leaks out through the defect. In this case, the barrier properties of the cell sheet are evaluated to be reduced as compared with a defect-free state of the cell sheet. Thus, the evaluation of the barrier properties of the cell sheet is carried out by detecting defects in the cell sheet.

Evaluation of the barrier properties of the cell sheet is carried out, for example, to examine the efficacy or side effects of a drug. That is, the efficacy or side effects of the drug can be examine by detecting defects on the cell sheet in a case where the drug and the cell sheet are brought into contact with each other. In addition, as another example, the evaluation of the barrier properties of the cell sheet is carried out by the manufacturer of the flow channel device in order to confirm that the cell sheet in the flow channel device is not defective in inspections of products before shipment. In addition, the evaluation of the barrier properties of the cell sheet is carried out in the development of a flow channel device 11 for checking whether or not the cell sheet conforms to the specifications and the like.

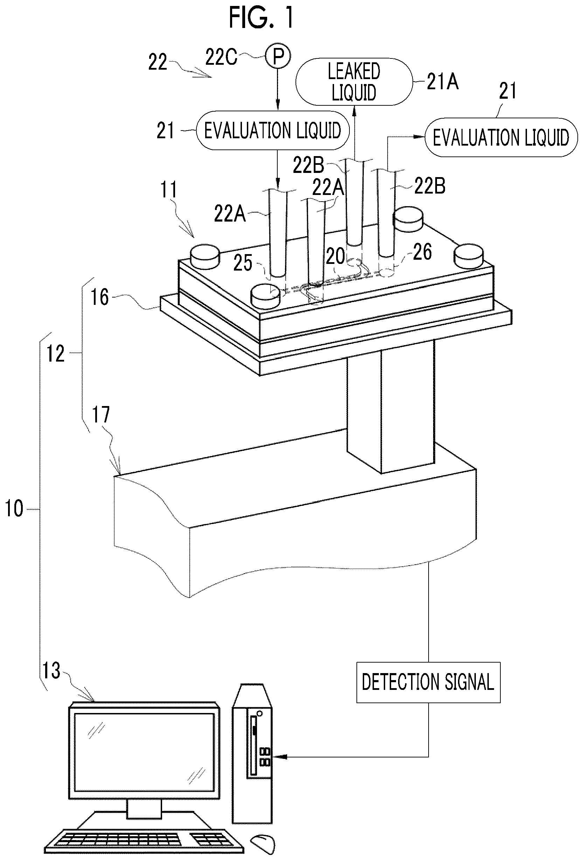

In JP2018-522586A, the method for evaluating the barrier properties of the cell sheet is as follows. First, the flow channel device described in JP2018-522586A has two flow channels, a first flow channel (perfusion channel) and a second flow channel (gel channel) (paragraph 0155, Fig. 1A, and Fig. 1B). The first flow channel and the second flow channel are partially in communication with each other, and a communicating portion therebetween is partitioned by a cell sheet.

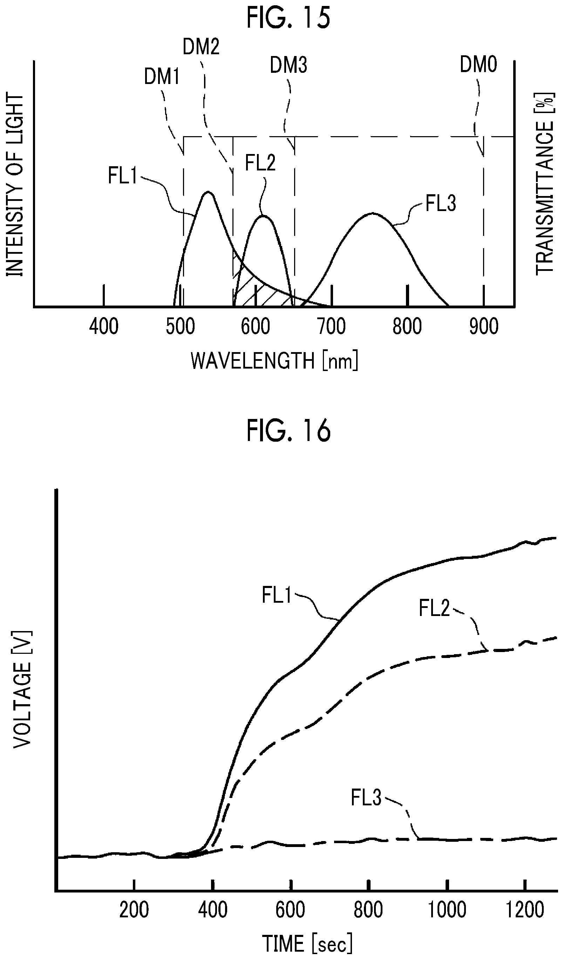

In the method described in JP2018-522586A, an evaluation liquid for evaluating the barrier properties of the cell sheet is injected into the first flow channel of such a flow channel device. A fluorescent probe is used as an example of the tracer in the evaluation liquid, and a culture medium is used as a solvent for the fluorescent probe. In a case where the cell sheet is not defective and has perfect barrier properties, the fluorescent probe will not leak out into the second flow channel. On the other hand, in a case where the cell sheet is defective, the fluorescent probe will flow out into the second flow channel. Such a leakage state is observed using a fluorescence microscope (paragraph 0155, Fig. 1 to Fig. 3). In addition, JP2018-522586A describes that a plurality of types of tracers (fluorescent probes) having different sizes may be used as tracers (fluorescent probes) (paragraph 0155, Fig. 16).

### SUMMARY OF THE INVENTION

### Technical Problem

There are various types of defects in the cell sheet, such as a defect caused by the shedding of some cells, such as dead cells, from the cell sheet, a defect caused by the destruction of tight junctions between adjacent cells, and a defect caused by the partial peeling of the cell sheet. In addition, the size of these defects also varies. For example, the defect caused by the destruction of tight junctions is on the order of 1 nanometer (nm) to several tens of nm, whereas the defect caused by the shedding of cells and the partial peeling of the cell sheet is on the order of 1 micrometer (µm) to several tens of µm.

In the evaluation of the barrier properties of the cell sheet, it is required not only to simply detect the presence or absence of defects but also to detect a more detailed state of the defects such as an approximate size of the defects occurred in the cell sheet and an approximate proportion of each of defects having different sizes. This is because the detection of such a more detailed defect state makes it possible to evaluate the barrier properties of the cell sheet in more detail as compared with the case where the presence or absence of a defect is simply detected.

As described above, JP2018-522586A describes that a plurality of types of tracers having different sizes may be used as the tracers (fluorescent probes). However, JP2018-522586A does not clearly describe the purpose of use and the method of use of a plurality of types of tracers having different sizes.

It is conceivable to detect defects of a plurality of types of sizes by using a plurality of types of tracers having different sizes as follows. That is, first, a large size defect is detected using an evaluation liquid containing a tracer having a relatively large size (large size tracer). The large size tracer penetrates through only a large size defect. Therefore, leakage of the large size tracer indicates the presence of the large size defect.

In addition, in a case where there is no large size defect on the cell sheet, a small size defect is detected using an evaluation liquid containing a small size tracer having a relatively small size. In a case where there is no large size defect on the cell sheet, the small size defect can be detected by gradually reducing the size of the tracer.

It is possible to detect what size defect has occurred in the cell sheet by using a plurality of types of evaluation liquids containing tracers having different sizes in this way and detecting defects while exchanging these evaluation liquids in order in the flow channel device.

However, such a method of properly using a plurality of types of evaluation liquids containing tracers having different sizes has a problem that it takes time and effort because the evaluation liquids need to be exchanged.

An object of the technique of the present disclosure is to provide a measurement device, an operation program for a measurement device, and a measurement method, which are used for evaluating barrier properties of a cell sheet and are capable of detecting a plurality of types of defects having different sizes occurring in the cell sheet without spending time and effort as compared with the related art technique.

### Solution to Problem

In order to achieve the foregoing object, a measurement device according to the technique of the present disclosure includes a mounting unit in which a flow channel device is attachably and detachably mounted, the flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion; and a detection unit that optically distinguishes and detects a leakage state of each of a plurality of types of tracers having different sizes and leaking out from the first flow channel into the second flow channel through a defect occurring in the cell sheet in a case where an evaluation liquid is fed into the first flow channel, the evaluation liquid being used for evaluation of barrier properties of the cell sheet and containing the plurality of types of tracers in a mixed state.

It is preferable that each of the plurality of types of tracers is a fluorescent tracer which emits fluorescence having a different wavelength range, and the detection unit detects a plurality of types of fluorescence emitted by each of the plurality of types of tracers.

The detection unit preferably includes an excitation light source for irradiating each of the plurality of types of tracers with excitation light that excites the fluorescence, an optical filter for separating the plurality of types of fluorescence, and a light receiving section for receiving the fluorescence separated by the optical filter.

It is preferable that the number of the excitation light source is one, and the light receiving section is provided for each of the plurality of types of fluorescence.

It is preferable that the excitation light source is on a downstream side in a flow direction of the tracer leaking out from the communicating portion in the flow channel device mounted on the mounting unit, and irradiates a position where light is incident only on the second flow channel of the first flow channel and the second flow channel with excitation light.

The leakage state preferably includes a time change in a leakage amount.

It is preferable to have a data processing unit that carries out data processing on detection signals output by the detection unit.

The data processing unit preferably has a signal processing unit that executes at least one of a correction process for correcting a dark current of the detection unit, a removal process for removing an overlapping portion of a wavelength range of a plurality of types of light from the detection signal corresponding to each light, or a conversion process for converting the detection signal output as a voltage signal into an intensity of light.

The data processing unit preferably has a concentration derivation unit for deriving a concentration of each of the plurality of types of tracers leaked out from the first flow channel to the second flow channel based on the detection signal output by the detection unit.

The data processing unit preferably has a defect state derivation unit for deriving a defect state occurring in the cell sheet based on the concentration of each of the plurality of types of tracers.

It is preferable to have an output control unit for outputting the leakage state detected by the detection unit to a display in real time.

The cell sheet is cultured on a support, and the support is preferably a permeable membrane having pores having a diameter of 1 µm or more.

The particle size of each of the plurality of types of tracers is preferably within a range of 1 nm or more and 100 µm or less.

The plurality of types of tracers preferably include a tracer having a particle size on the order of 1 nm to several hundreds of nm and a tracer having a particle size on the order of 1 µm to several tens of µm.

It is preferable to have a supply mechanism for supplying the evaluation liquid to the first flow channel.

An operation program for a measurement device according to the technique of the present disclosure is an operation program for a measurement device for evaluating barrier properties of a cell sheet using a flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion, the operation program causing a computer to function as an acquisition unit for acquiring a detection signal from a detection unit that optically distinguishes and detects a leakage state of each of a plurality of types of tracers having different sizes and leaking out from the first flow channel into the second flow channel through a defect occurring in the cell sheet in a case where an evaluation liquid is fed into the first flow channel, the evaluation liquid being used for evaluation of the barrier properties of the cell sheet and containing the plurality of types of tracers in a mixed state; and a processing unit for executing an output control process that outputs the leakage state based on the acquired detection signal.

A measurement method according to the technique of the present disclosure uses a flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion; and includes a liquid feeding step of feeding an evaluation liquid to the first flow channel, the evaluation liquid being used for evaluating barrier properties of the cell sheet and containing a plurality of types of tracers having different sizes in a mixed state; and a detection step of optically distinguishing and detecting a leakage state of each of the plurality of types of tracers leaking out from the first flow channel into the second flow channel through a defect occurring in the cell sheet.

### Effects of Invention

According to the technique of the present disclosure, it is possible to provide a measurement device, an operation program for a measurement device, and a measurement method, which are used for evaluating the barrier properties of a cell sheet and are capable of detecting a plurality of types of defects having different sizes occurring in the cell sheet without spending time and effort as compared with the related art technique.

### BRIEF DESCRIPTION OF THE DRAWINGS

Fig. 1 is an overall configuration diagram of a device for evaluating barrier properties of a cell sheet.
Fig. 2 is an exploded perspective view of a flow channel device.
Fig. 3 is a transverse cross-sectional view of a flow channel device.
Fig. 4 is a vertical cross-sectional view of the flow channel device.
Fig. 5 is a plan view showing an overlap between a first flow channel and a second flow channel.
Fig. 6 is an explanatory diagram of a permeable membrane.
Fig. 7 is an explanatory diagram of a pore diameter of the permeable membrane and a particle size of a tracer.
Fig. 8 is an explanatory diagram of a cell sheet formed on the permeable membrane.
Fig. 9 is an explanatory diagram of defects occurring in the cell sheet.
Fig. 10 is an explanatory diagram of a state in which an evaluation liquid is fed to the first flow channel. Fig. 10A is an explanatory diagram showing the flow of the evaluation liquid in a case where there is no defect in the cell sheet, and Fig. 10B is an explanatory diagram showing the flow of the evaluation liquid in a case where the cell sheet is defective.
Fig. 11 is an external perspective view of a detection unit.
Fig. 12 is an explanatory diagram showing a configuration of an optical component of the detection unit. Fig. 12A is a plan view and Fig. 12B is a side view.
Fig. 13 is an explanatory diagram showing characteristic features of a filter. Fig. 13A shows characteristic features of a first filter, Fig. 13B shows characteristic features of a second filter, and Fig. 13C shows characteristic features of a third filter.
Fig. 14 is an explanatory diagram showing a wavelength of fluorescence emitted by each of tracers. Fig. 14A shows a wavelength of fluorescence of a first tracer, Fig. 14B shows a wavelength of fluorescence of a second tracer, and Fig. 14C shows a wavelength of fluorescence of a third tracer.
Fig. 15 is an explanatory diagram showing the relationship between the wavelength of fluorescence emitted by each of tracers and the characteristic features of each of filters.
Fig. 16 is an explanatory diagram of a detection signal output by each of light receiving sections.
Fig. 17 shows a schematic configuration diagram of a computer that functions as a control device.
Fig. 18 is an explanatory diagram of a functional block of the control device.
Fig. 19 is an explanatory diagram of a signal process.
Fig. 20 is an explanatory diagram of a concentration derivation process.
Fig. 21 is an explanatory diagram showing a correspondence relationship of each of tracers with a size of a defect through which each of tracers can penetrate.
Fig. 22 is a graph showing a relationship between a total area of defects on the cell sheet and an indicator (concentration) of a leakage amount of each of tracers.
Fig. 23 is an explanatory diagram of an outline of a defect state derivation process.
Fig. 24 is an explanatory diagram of details of the defect state derivation process.
Fig. 25 is an explanatory diagram of a measurement result display screen.
Fig. 26 is a flowchart showing a procedure of a measurement process.
Fig. 27 is a flowchart showing the procedure of the measurement process of Comparative Example.
Fig. 28 is an explanatory diagram showing an example of forming cell sheets on both surfaces of the permeable membrane.
Fig. 29 is an explanatory diagram showing a state in which the cell sheet is defective in the example of Fig. 28.
Fig. 30 is an explanatory diagram showing an example of a detection unit having a rotation filter unit.
Fig. 31 is an explanatory diagram showing characteristic features of each filter of the rotation filter unit of Fig. 30.

### DESCRIPTION OF EMBODIMENTS

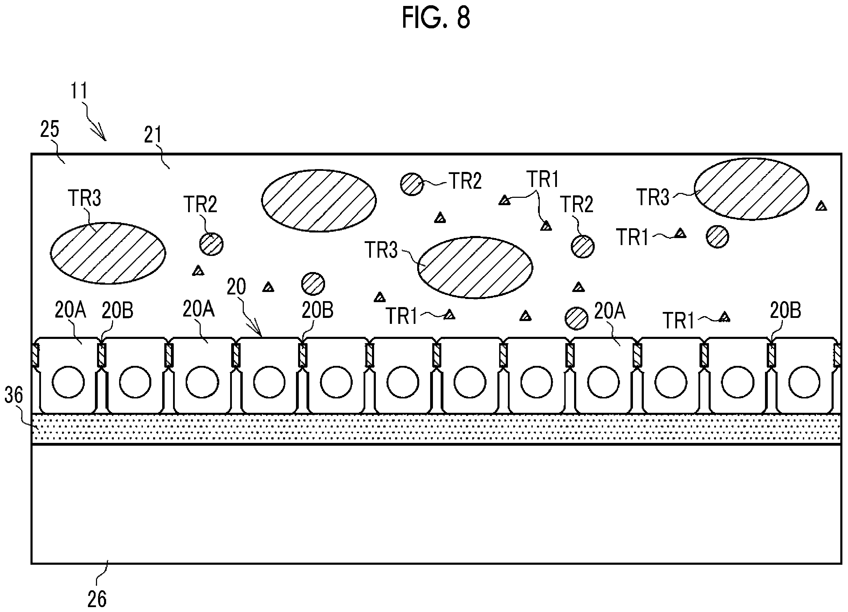

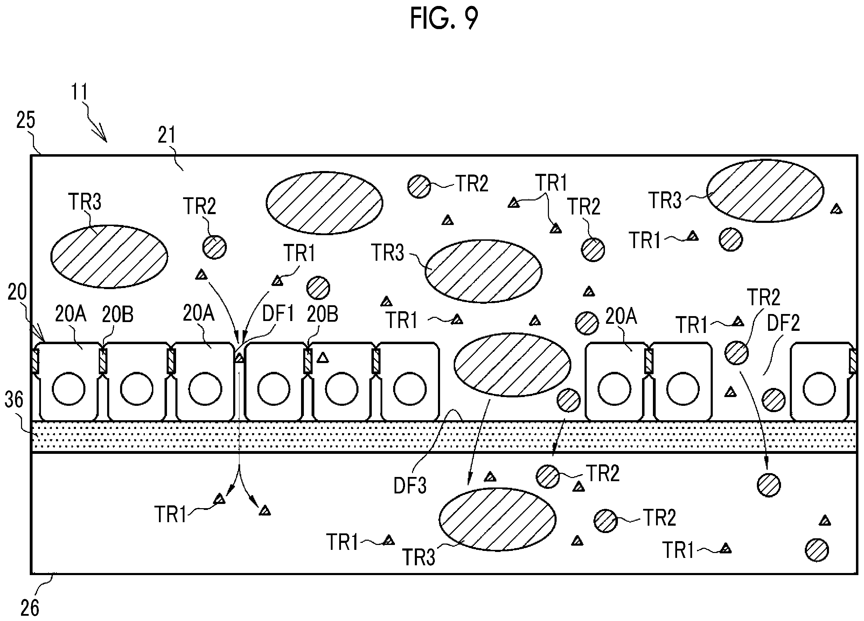

As shown as an example in Fig. 1, a measurement device 10 is used for evaluating the barrier properties of a cell sheet 20 (see also Fig. 8 and Fig. 9) formed in a flow channel device 11. The cell sheet 20 is formed by seeding and culturing in the flow channel device 11. The flow channel device 11 on which the cell sheet 20 is formed is attachably and detachably set in the measurement device 10. Then, in order to evaluate the barrier properties of the cell sheet 20 in the set flow channel device 11, the measurement device 10 measures the data as an indicator of the evaluation. Data that can be used as an indicator for evaluating the barrier properties include, for example, the state of defects including the proportion of defects having different sizes, in addition to the presence or absence of defects that occur in the cell sheet 20.

As an example, the measurement device 10 is used by a manufacturer of the flow channel device 11 to evaluate the barrier properties of the cell sheet 20 formed in the flow channel device 11 in a case of developing the flow channel device 11.

The measurement device 10 includes a detection device 12 and a control device 13. The detection device 12 includes a mounting unit 16 and a detection unit 17. The flow channel device 11 is attachably and detachably mounted on the mounting unit 16.

As will be described in detail later, the detection unit 17 optically detects a leakage state of a fluorescent tracer TR (see Fig. 8 and Fig. 9) that leaks out through a defect occurring in the cell sheet 20, in a case where an evaluation liquid 21 for evaluating the barrier properties of the cell sheet 20 is fed to the flow channel device 11. The evaluation liquid 21 is a liquid in which the fluorescent tracer TR is contained in a solvent such as a buffer. The tracer is a substance used to track the flow of a fluid such as a liquid, or a specific substance. The fluorescent tracer TR refers to a tracer that emits fluorescence. Specifically, a fluorescent substance itself, a substance labeled with the fluorescent substance, or the like is used as the fluorescent tracer TR. The detection unit 17 detects the leakage state by detecting the fluorescence FL emitted by the leaked fluorescent tracer TR.

The control device 13 controls the operation of the detection unit 17. Further, the control device 13 acquires a detection signal output by the detection unit 17, and carries out data processing on the acquired detection signal. As a result, various measurement results based on the detection signal are output. The control device 13 is composed of, for example, a computer.

The measurement device 10 further includes a supply mechanism 22 for supplying a liquid such as the evaluation liquid 21 to the flow channel device 11 mounted on the mounting unit 16. The supply mechanism 22 includes a supply tube 22A, a discharge tube 22B, and a pump 22C. The supply tube 22A and the discharge tube 22B are attached to the flow channel device 11 mounted on the mounting unit 16.

The flow channel device 11 includes two flow channels, a first flow channel 25 and a second flow channel 26. Two supply tubes 22A and two discharge tubes 22B are provided, and the supply tube 22A and the discharge tube 22B are attached to each of the first flow channel 25 and the second flow channel 26.

The pump 22C is connected to the supply tube 22A attached to the first flow channel 25. The pump 22C is, for example, a syringe pump. The pump 22C supplies the evaluation liquid 21 to the first flow channel 25 through the supply tube 22A. As will be described later, the first flow channel 25 and the second flow channel 26 are partially in communication with each other, and the cell sheet 20 partitions the first flow channel 25 and the second flow channel 26 at this communicating portion.

In a case where the cell sheet 20 is not defective, the communicating portion between the first flow channel 25 and the second flow channel 26 is sealed by the cell sheet 20. Therefore, in a case where the cell sheet 20 is not defective, the fluorescent tracer TR contained in the evaluation liquid 21 does not leak out to the second flow channel 26 in a case where the evaluation liquid 21 is fed to the first flow channel 25. The evaluation liquid 21 supplied to the first flow channel 25 is discharged from the discharge tube 22B after passing through the first flow channel 25.

On the other hand, in a case where the cell sheet 20 is defective, the fluorescent tracer TR in the evaluation liquid 21 supplied to the first flow channel 25 leaks out to the second flow channel 26 through the defect. In a case where the cell sheet 20 is defective, a leaked liquid 21A, which is a part of the evaluation liquid 21, flows into the second flow channel 26. The leaked liquid 21A contains the fluorescent tracer TR. The leaked liquid 21A is discharged from the discharge tube 22B after passing through the second flow channel 26.

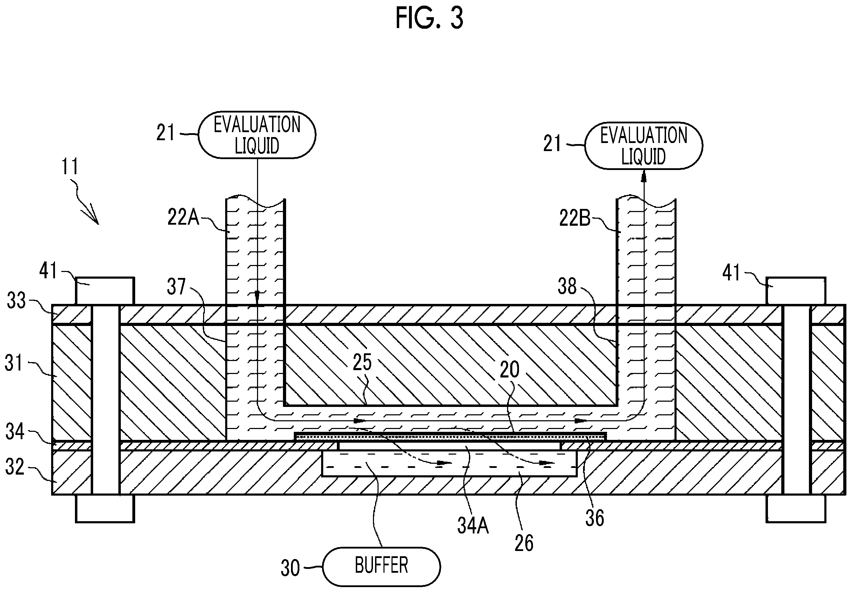

The supply tube 22A attached to the second flow channel 26 is used to supply a buffer 30 (see Fig. 3 and the like) to the second flow channel 26. For example, Tris buffered saline (TBS) or Hanks' balanced salt solution (HBSS) is used as the buffer 30. The buffer 30 is the same as the buffer used as the solvent of the evaluation liquid 21 described above, for example.

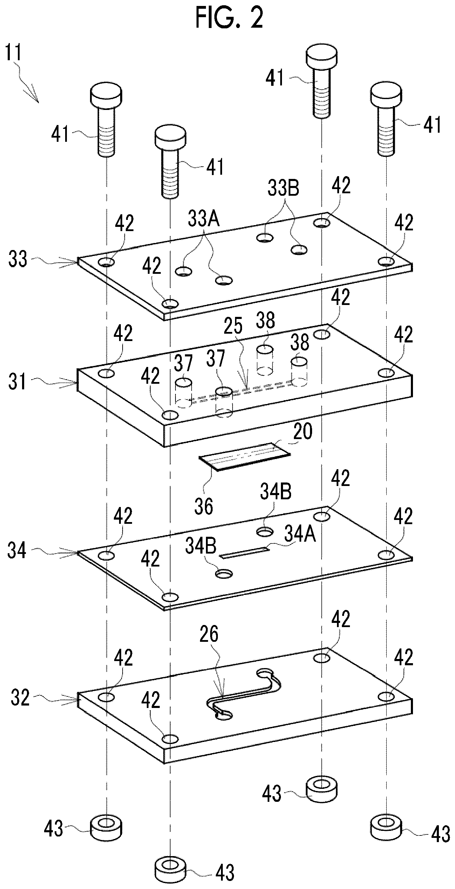

As shown in Fig. 2, the flow channel device 11 includes a first flow channel member 31, a second flow channel member 32, a holding plate 33, a reinforcing plate 34, and a permeable membrane 36. In the first flow channel member 31, the first flow channel 25 is formed on the lower surface of the lower side thereof in Fig. 2. In the second flow channel member 32, the second flow channel 26 is formed on the upper surface of the upper side thereof in Fig. 2. The first flow channel member 31 and the second flow channel member 32 are assembled in such a manner that the first flow channel 25 and the second flow channel 26 are arranged so as to face each other.

The first flow channel member 31 is made of, for example, an elastic transparent resin material such as polydimethylsiloxane (PDMS), and the second flow channel member 32 is made of, for example, a stiff transparent resin material such as cycloolefin polymer (COP).

The first flow channel member 31 is provided with an injection passage 37 and a discharge passage 38 connected to the first flow channel 25. In addition, the first flow channel member 31 is also formed with an injection passage 37 and a discharge passage 38 connected to the second flow channel 26. That is, two injection passages 37 and two discharge passages 38 are provided corresponding to the first flow channel 25 and the second flow channel 26, respectively. The injection passage 37 and the discharge passage 38 are through-passages that penetrate through the first flow channel member 31 in the vertical direction, that is, in the thickness direction.

The holding plate 33 is arranged on the upper surface of the first flow channel member 31. The holding plate 33 includes an injection port 33A and a discharge port 33B formed at positions corresponding to the injection passage 37 and the discharge passage 38 of the first flow channel member 31, respectively. The supply tube 22A and the discharge tube 22B are connected to the injection port 33A and the discharge port 33B, respectively.

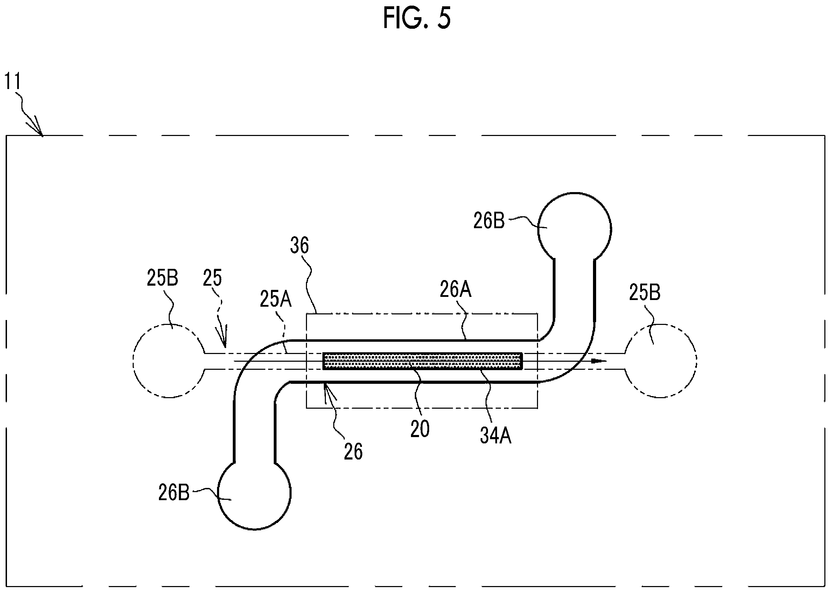

The reinforcing plate 34 reinforces the stiffness of the flow channel device 11. The reinforcing plate 34 is arranged between the first flow channel member 31 and the second flow channel member 32. In the reinforcing plate 34, a slit 34A formed elongated along the direction in which the evaluation liquid 21 flows in the first flow channel 25 is formed at a position corresponding to the first flow channel 25 and the second flow channel 26. The slit 34A is arranged at the communicating portion between the first flow channel 25 and the second flow channel 26. In addition, the reinforcing plate 34 is formed with openings 34B. The openings 34B are arranged at positions corresponding to connecting portions 26B (see Fig. 5) formed at both ends of the second flow channel 26 of the second flow channel member 32. In addition, the openings 34B connect to the injection passage 37 and the discharge passage 38 formed for the second flow channel 26 in the first flow channel member 31.

The permeable membrane 36 is arranged between the first flow channel member 31 and the reinforcing plate 34, and is arranged at a position corresponding to the slit 34A. The permeable membrane 36 functions as a support for the cell sheet 20, and the cell sheet 20 is seeded and cultured on the permeable membrane 36. In addition, the permeable membrane 36 is permeable to the fluorescent tracer TR (see Fig. 8, Fig. 9, and the like) contained in the evaluation liquid 21.

Screw holes 42 for inserting bolts 41 are formed in each of the holding plate 33, the first flow channel member 31, the reinforcing plate 34, and the second flow channel member 32. The holding plate 33, the first flow channel member 31, the reinforcing plate 34, and the second flow channel member 32 are laminated in this order to be assembled as the flow channel device 11. Then, the flow channel device 11 is fastened by bolts 41 and nuts 43 in the assembled state.

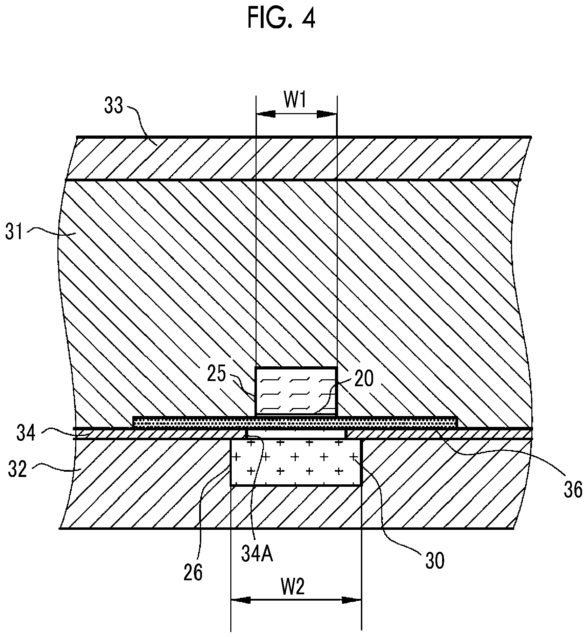

As shown in Fig. 3 to Fig. 5, in a state where the flow channel device 11 is assembled, linear portions 25A and 26A (see Fig. 5) of the first flow channel 25 and the second flow channel 26 face each other. Fig. 3 is a transverse cross-sectional view of the flow channel device 11, Fig. 4 is a vertical cross-sectional view of the flow channel device 11, and Fig. 5 is a plan view showing an overlap between the first flow channel 25 and the second flow channel 26.

As shown in Fig. 4, a width W1 of the linear portion 25A of the first flow channel 25 is, for example, about 200 µm, and a width W2 of the linear portion 26A of the second flow channel 26 is, for example, about 400 µm. As shown in Fig. 5, a part of the linear portion 25A and a part of the linear portion 26A overlap. The slit 34A is arranged at this overlap position, and therefore the portion corresponding to the slit 34A serves as a communicating portion of each of the first flow channel 25 and the second flow channel 26. In addition, since the first flow channel 25 and the second flow channel 26 have a flow channel having a width on the order of microns, the flow channel device 11 is also called a micro flow channel device or a microfluidic device.

The cell sheet 20 is formed on the surface of the permeable membrane 36 on the side of the first flow channel 25. The cell sheet 20 is formed in the communicating portion between the linear portion 25A and the linear portion 26A to partition the first flow channel 25 and the second flow channel 26 in the linear portions 25A and 26A.

In Fig. 3, as shown by the arrow of the alternate long and two short dashes line, in a case where the cell sheet 20 is defective, a part of the evaluation liquid 21 in the first flow channel 25 passes through the permeable membrane 36 and the slit 34A and consequently the leaked liquid 21A containing the fluorescent tracer TR (Fig. 8, Fig. 9, and the like) leaks into the second flow channel 26.

In Fig. 5, connecting portions 25B connected to the injection passage 37 and the discharge passage 38 are formed at the upstream end and the downstream end of the linear portion 25A of the first flow channel 25. In addition, connecting portions 26B connected to the injection passage 37 and the discharge passage 38 are formed at the upstream end and the downstream end of the linear portion 26A of the second flow channel 26.

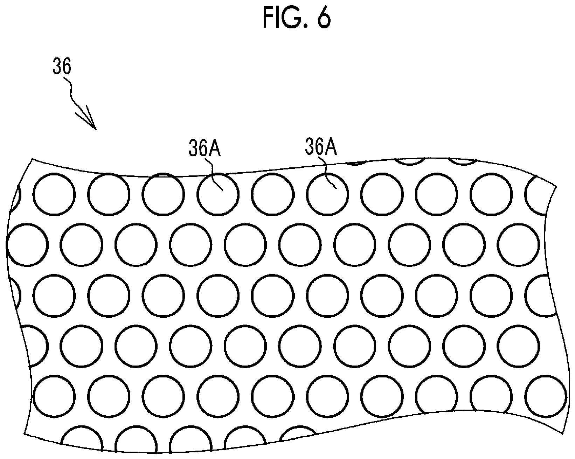

As shown in Fig. 6, the permeable membrane 36 is a porous membrane in which a large number of pores 36A are formed. The permeable membrane 36 is made of, for example, a hydrophobic polymer that is soluble in a hydrophobic organic solvent. Examples of the hydrophobic polymer include polymers such as polystyrene, polyacrylate, polymethacrylate, polyacrylamide, polycarbonate, and polypropylene. These polymers may be used alone or in admixture of two or more thereof. In addition, the material of the permeable membrane 36 is not limited to the hydrophobic polymer, and various materials can be selected from the viewpoint of cellular adhesiveness and the like.

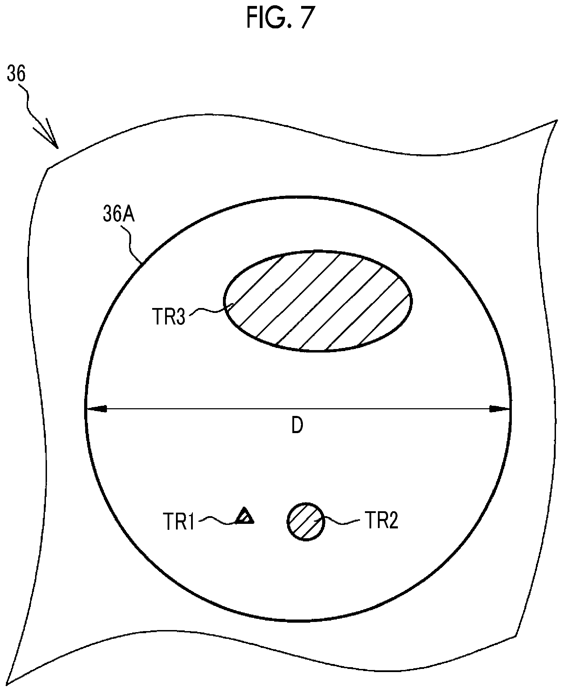

As shown in Fig. 7, a diameter D of the pore 36A of the permeable membrane 36 is a size permeable to the fluorescent tracer TR contained in the evaluation liquid 21. In this example, the diameter D is 1 µm or more. The permeable membrane 36 of this example is a honeycomb film in which a plurality of pores 36A are arranged in a honeycomb arrangement. Of course, a film in which a plurality of pores 36A are arranged in a square arrangement may be used. The diameter D of the pore 36A is, for example, about 5 µm to about 20 µm, and in this example, about 20 µm. The opening ratio is, for example, about 20% to about 50%, and in this example, about 50%. The opening ratio may be 50% or more.

The evaluation liquid 21 contains a plurality of types of fluorescent tracers TR having different sizes, specifically, different particle sizes. In this example, three types of fluorescent tracers TR1, TR2, and TR3 having different sizes are contained. In the present specification, in a case where it is necessary to distinguish each of the three types of fluorescent tracers TR1 to TR3 having different sizes, a reference numeral having a number added to the reference numeral TR, such as TR1 to TR3, is used; but, in a case where it is not necessary to distinguish between the three types of fluorescent tracers TR having different sizes, the reference numeral TR is simply used with omission of the number.

Each of the fluorescent tracers TR1 to TR3 is a tracer that emits fluorescence upon irradiation with excitation light. As will be described later, the wavelength range of fluorescence is different for each of the three types of fluorescent tracers TR1 to TR3 having different sizes.

In addition, the size (particle size) of each of the three types of fluorescent tracers TR1 to TR3 is, for example, within a range of 1 nm or more and 100 µm or less. In this example, since the diameter D of the pore 36A is 20 µm, a fluorescent tracer TR of less than 20 µm is used.

Specifically, the fluorescent tracer TR1 has a size of about several nm to several tens of nm, which is the smallest among the three types of fluorescent tracers TR. The fluorescent tracer TR3 has a size of about 6 µm to 8 µm, which is the largest among the three types of fluorescent tracers TR. The fluorescent tracer TR2 has a size of about 200 nm to 300 nm, which is an intermediate size between the fluorescent tracer TR1 and the fluorescent tracer TR2. As described above, in this example, the fluorescent tracer TR1 having a size (particle size) on the order of 1 nm to several tens of nm, the fluorescent tracer TR2 having a size (particle size) on the order of several hundreds of nm, and the fluorescent tracer TR3 having a size (particle size) on the order of 1 µm to 10 µm are included as the fluorescent tracers TR1 to TR3 having different sizes.

For example, fluorescent molecules having a size (particle size) of 1 nm, dextran having various sizes (particle sizes) of several nm to 100 nm, and fluorescent beads having various sizes (particle sizes) of 10 nm to 100 µm can be used as the fluorescent tracer TR. Examples of the dextran include fluorescein, Alexa, and tetramethylrhodamine. In addition, red blood cells labeled with a fluorescent dye may be used as the fluorescent tracer TR. The size (particle size) of red blood cells is from about 6 µm to about 8 µm. The size combination of the fluorescent tracers TR contained in the evaluation liquid 21 is appropriately selected from these fluorescent tracers.

As shown in Fig. 8, the cell sheet 20 is arranged in a state where a plurality of cells 20A are bound on the permeable membrane 36. The cell sheet 20 is formed, for example, as follows. First, at the time of producing the flow channel device 11, a culture medium and the cells 20A are supplied to the permeable membrane 36 through the first flow channel 25. As a result, the cells are cultured on the permeable membrane 36, and a plurality of cells 20A are arranged on the permeable membrane 36 in such a manner that the cells are bound to each other. The cultured cell sheet 20 is adsorbed onto the permeable membrane 36.

The first flow channel 25 of the flow channel device 11 is simulated as, for example, a blood vessel. In this case, for example, endothelial cells constituting inner walls of blood vessels are formed as the cell sheet 20. Tight junctions 20B are formed between the cells 20A in the endothelial cells. Tight junctions are proteins that are expressed in a case where the cells 20A adhere to each other. In a cell sheet having tight junctions such as the cell sheet 20, no gaps are formed between the cells 20A. The cell sheet 20 having such tight junctions 20B has high barrier properties in a case where no defect has occurred.

As shown in Fig. 9, there are various types of defects DF in the cell sheet 20, such as a defect DF1, which is caused by the destruction of tight junctions 20B between adjacent cells 20A; a defect DF2, which is caused by the shedding of some cells 20A, such as dead cells, from the cell sheet 20; and a defect DF3, which is caused by the partial peeling of the cell sheet 20. The defect DF3 due to the partial peeling of the cell sheet 20 is caused by, for example, an incomplete adsorption portion of the cells 20A onto the permeable membrane 36 coming into contact with the liquid flowing through the first flow channel 25.

In a case where the cell sheet 20 is provided in the fine first flow channel 25 as in the flow channel device 11, the fluid resistance of the liquid flowing in the first flow channel 25 is large. Therefore, in a case where the liquid comes into contact with the cell sheet 20, the cell sheet 20 is likely to be peeled off.

The sizes of these defects DF1 to DF23 vary. For example, the size of the defect DF1 caused by the destruction of tight junctions 20B is on the order of several nm to several tens of nm; whereas the size of the defect DF2 caused by the shedding of cells 20A is on the order of several hundreds of nm, and the size of the defect DF3 caused by the partial peeling of the cell sheet 20 is on the order of 1 µm to 100 µm. Of the defects DF1 to DF3, the defect DF1 has the smallest size (small size). The defect DF3 has the largest size (large size). The defect DF2 has a medium size. Here, similar to the case of the fluorescent tracers TR, the defects DF1 to DF3 are also simply referred to as defect DF without adding a number, in a case of generically referring to those defects DF1 and DF3 without distinguishing therebetween.

The small size defect DF1 has a size permeable to the small size fluorescent tracer TR1, the medium size defect DF2 has a size permeable to the medium size fluorescent tracer TR2, and the large size defect DF3 has a size permeable to the large size fluorescent tracer TR3.

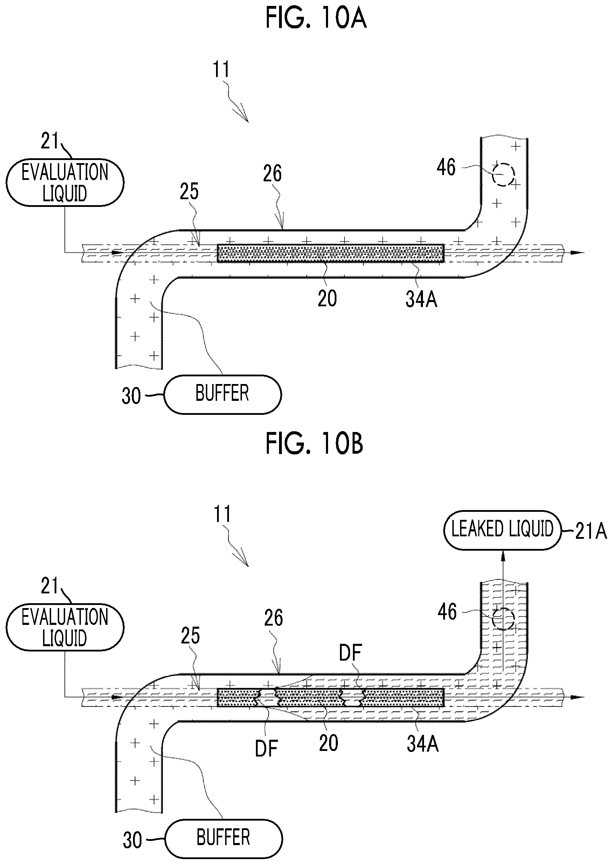

As shown in Fig. 10, in a case where the evaluation liquid 21 in which the fluorescent tracers TR1 to TR3 of three different sizes are in a mixed state is flowed through the first flow channel 25, there is no leakage of the leaked liquid 21A containing fluorescent tracers TR1 to TR3 into the second flow channel 26 in a case where the cell sheet 20 is free of the defect DF, as shown in Fig. 10A. On the other hand, as shown in Fig. 10B, in a case where there is an occurrence of the defect DF in the cell sheet 20, the leaked liquid 21A containing at least the small size fluorescent tracer TR1 out of three sizes of fluorescent tracers TR1 to TR3 leaks out according to the size of the defect DF.

Reference numeral 46 indicates a detection position at which the detection unit 17 detects the leakage state of the leaked liquid 21A containing the fluorescent tracer TR. The detection position 46 is on the downstream side in the flow direction of the fluorescent tracer TR leaking out from the position corresponding to the slit 34A which is the communicating portion, and is set at the position where the excitation light is incident only on the second flow channel 26 of the first flow channel 25 and the second flow channel 26.

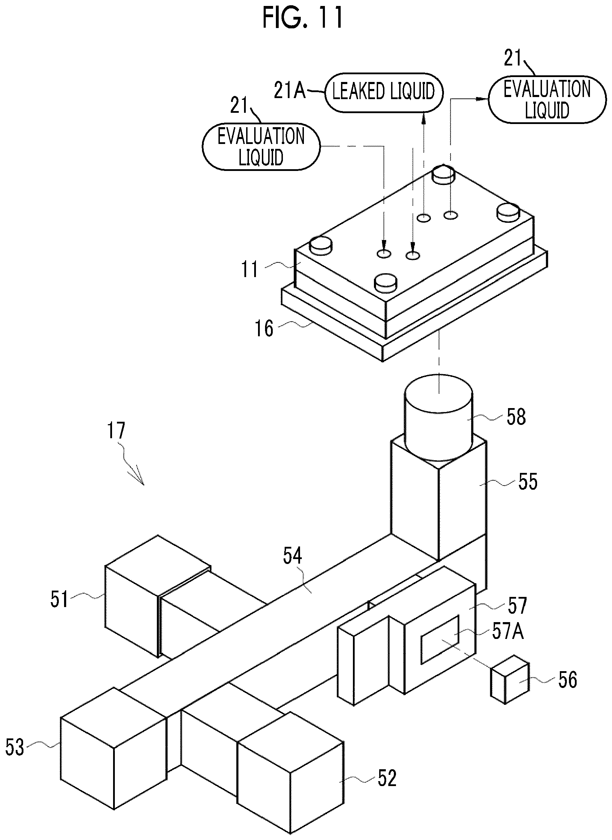

As will be described later, the detection unit 17 has an excitation light source 56 (see Fig. 11) that irradiates a target site with excitation light. That is, the excitation light source 56 is on the downstream side in the flow direction of the evaluation liquid 21 leaking out from the slit 34A corresponding to the communicating portion and the defect DF of the cell sheet 20 in the flow channel device 11 mounted on the mounting unit 16, and irradiates a position where the light is incident only on the second flow channel 26 of the first flow channel 25 and the second flow channel 26 with the excitation light.

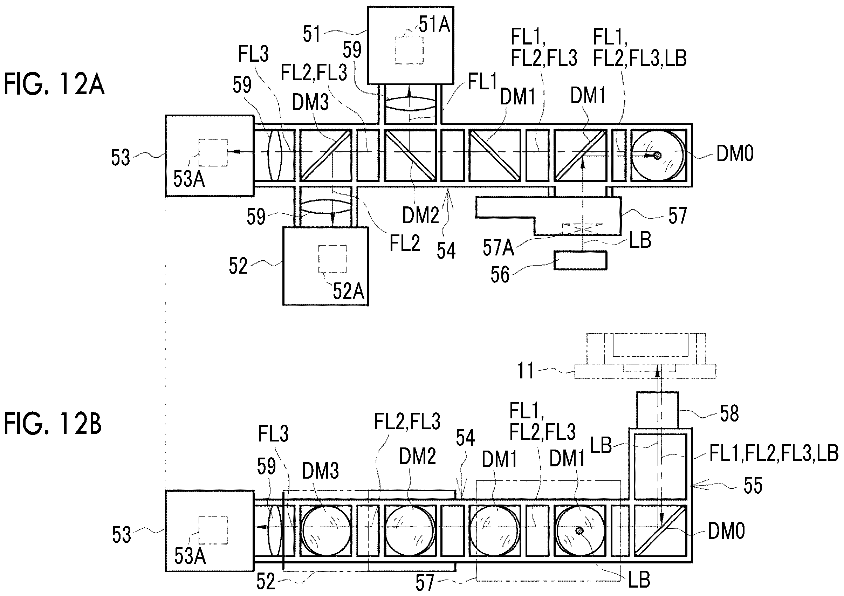

As shown in Fig. 11, the detection unit 17 includes a first detector 51, a second detector 52, a third detector 53, a first lens barrel unit 54, a second lens barrel unit 55, an excitation light source 56, a shutter unit 57, and an objective lens 58. The excitation light source 56 is, for example, a laser light source such as a semiconductor laser, and emits excitation light LB (see Fig. 12) having a central wavelength of, for example, 476 nm as excitation light.

The shutter unit 57 is arranged in front of the excitation light source 56 in the irradiation direction. The shutter unit 57 moves a shutter 57A, which shields the excitation light, between the open position and the closed position. The shutter 57A is, for example, an electric shutter that operates electrically. In a case where the shutter 57A is in the open position, the excitation light LB is incident on the first lens barrel unit 54. On the other hand, in a case where the shutter 57A is in the closed position, the excitation light LB is shielded by the shutter 57A, and therefore the incidence of the excitation light LB is prohibited.

Fig. 12 mainly shows the configuration of optical components in the first lens barrel unit 54. Fig. 12A is a plan view of the inside of the first lens barrel unit 54 as viewed from the plane. Fig. 12B is a side view of the inside of the first lens barrel unit 54 as viewed from the side. Optical components such as dichroic mirrors DM0 to DM3 and a condenser lens 59 are provided in the first lens barrel unit 54. The dichroic mirrors DM0 to DM3 are optical filters that reflect or transmit light in a specific wavelength range.

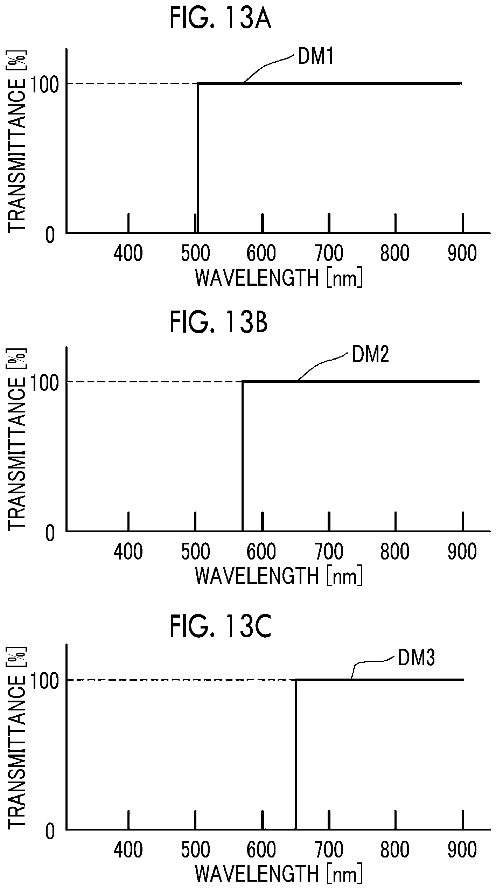

In Fig. 13, as shown in Fig. 13A, the dichroic mirror DM1 has transmission properties of reflecting light having a wavelength of shorter than 505 nm and transmitting light having a wavelength of 505 nm or longer. As shown in Fig. 13B, the dichroic mirror DM2 has transmission properties of reflecting light having a wavelength of shorter than 567 nm and transmitting light having a wavelength of 567 nm or longer. As shown in Fig. 13C, the dichroic mirror DM3 has transmission properties of reflecting light having a wavelength of shorter than 650 nm and transmitting light having a wavelength of 650 nm or longer.

Although not shown in Fig. 13, the dichroic mirror DM0 has transmission properties of reflecting light having a wavelength of shorter than 900 nm and transmitting light having a wavelength of 900 nm or longer (see Fig. 15). As the dichroic mirror DM0, a reflective mirror that exhibits specular reflection may be used because a wavelength selection system is not required for the fluorescence FL1 to FL3 and LB. However, in a case where the reflective mirror is used, infrared light in a wavelength range of 900 nm or longer is also reflected. In a case where infrared light is incident on the first detector 51 to the third detector 53, this causes a temperature rise, which is not preferable. Therefore, the dichroic mirror DM0 is used instead of the reflective mirror.

As shown in Fig. 12A, the excitation light LB that passes through the shutter 57A and is incident on the first lens barrel unit 54 has a central wavelength of 476 nm, and is therefore reflected by the dichroic mirror DM1. The dichroic mirror DM1 reflects the excitation light LB toward the dichroic mirror DM0. The dichroic mirror DM0 reflects the incident excitation light LB toward the objective lens 58 arranged at the downstream end of the second lens barrel unit 55.

The objective lens 58 causes the excitation light LB to be incident on the detection position 46 (see Fig. 10) of the flow channel device 11 mounted on the mounting unit 16. The objective lens 58 collects the excitation light LB so that the spot diameter of the excitation light LB fits in the width W2 (400 nm in this example) of the second flow channel 26.

In a case where the fluorescent tracers TR1 to TR3 leak out into the second flow channel 26, the detection position 46 is irradiated with the excitation light LB, whereby the fluorescence FL1 to FL3 in each wavelength range emit light from each of the fluorescent tracers TR1 to TR3. In this example, there is only one excitation light source 56, and each of fluorescent tracers TR1 to TR3 emits the fluorescence FL1 to FL3 in different wavelength ranges by one type of excitation light LB.

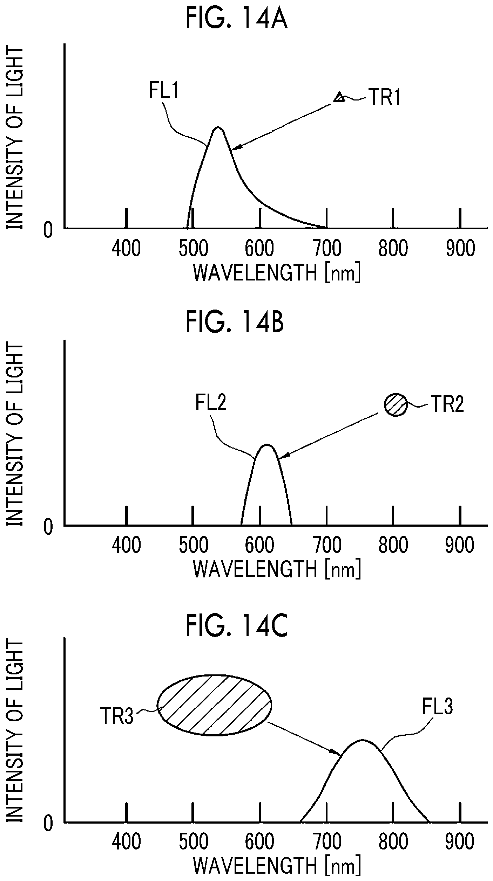

Fig. 14 shows an example of the wavelength range of the fluorescence FL1 to FL3 of each of fluorescent tracers TR1 to TR3. As shown in Fig. 14A, the fluorescence FL1 emitted by the small size fluorescent tracer TR1 has a wavelength range of about 500 nm to about 700 nm. As shown in Fig. 14B, the fluorescence FL2 emitted by the medium size fluorescent tracer TR2 has a wavelength range of about 570 nm to about 640 nm. As shown in Fig. 14C, the fluorescence FL3 emitted by the large size fluorescent tracer TR3 has a wavelength range of about 660 nm to about 850 nm.

As shown in Fig. 15, the dichroic mirror DM1 transmits all of these three types of fluorescence FL1 to FL3. The dichroic mirror DM2 transmits the fluorescence FL2 and FL3 out of three types of fluorescence FL1 to FL3. The dichroic mirror DM3 transmits the fluorescence FL3.

Referring back to Fig. 12, the fluorescence FL1 to FL3 emitted by each of the fluorescent tracers TR1 to TR3 are incident on the dichroic mirror DM0. In addition, the excitation light LB that is reflected by the flow channel device 11 and is re-incident on the objective lens 58 is also re-incident, in addition to the fluorescence FL1 to FL3.

The fluorescence FL1 to FL3 and the excitation light LB are reflected by the dichroic mirror DM0 and are incident on the dichroic mirror DM1. The dichroic mirror DM1 reflects the excitation light LB and transmits the fluorescence FL1 to FL3. In this example, the dichroic mirror DM1 has a two-sheet configuration, and the fluorescence FL1 to FL3 that have passed through the first dichroic mirror DM1 also pass through the second dichroic mirror DM1. The reason why the dichroic mirror DM1 is configured of two sheets is to surely cut laser light because the laser light having a high intensity of light is used as the excitation light LB.

A dichroic mirror DM2 is arranged after the dichroic mirror DM1. The dichroic mirror DM2 transmits the fluorescence FL2 and FL3 while reflecting the fluorescence FL1 toward the first detector 51. The reflected fluorescence FL1 is collected by the condenser lens 59. The collected light flux is incident on a light receiving section 51A in the first detector 51. In this way, the fluorescence FL1 is separated from the fluorescence FL2 and FL3 by the dichroic mirror DM2.

The fluorescence FL2 and FL3 that have passed through the dichroic mirror DM2 are incident on the dichroic mirror DM3. The dichroic mirror DM3 transmits the fluorescence FL3 while reflecting the fluorescence FL2 toward the second detector 52. The reflected fluorescence FL2 is collected by the condenser lens 59. The collected light flux is incident on a light receiving section 52A in the second detector 52. In this way, the fluorescence FL2 is separated from the fluorescence FL3 by the dichroic mirror DM3.

The fluorescence FL3 transmitted through the dichroic mirror DM3 is collected by the condenser lens 59. The collected light flux is incident on a light receiving section 53A in the third detector 53.

As described above, the dichroic mirrors DM2 and DM3 function as optical filters for separating each of the three types of fluorescence FL1 to FL3. Three light receiving sections 51A to 53A of the first detector 51 to the third detector 53 are provided for each of the fluorescent tracers TR1 to TR3 and the corresponding fluorescence FL1 to FL3. Each of the light receiving sections 51A to 53A of the first detector 51 to the third detector 53 receives each of the three types of fluorescence FL1 to FL3 separated by the dichroic mirrors DM2 and DM3 .

A photomultiplier is used as an example of each of the light receiving sections 51A to 53A of the first detector 51 to the third detector 53. Since the photomultiplier has a sensitivity about 100 times higher than that of a fluorescence microscope, a detection signal having a high signal/noise (S/N) ratio can be obtained. Each of the first detector 51 to the third detector 53 outputs a voltage signal (voltage [V: volt]) corresponding to the intensity of light of each of the fluorescence FL1 to FL3 as a detection signal.

Fig. 16 shows an example of the detection signal of each of the fluorescence FL1 to FL3 output by each of the detectors 51 to 53. In Fig. 16, the lateral axis is time, and the graph shown in Fig. 16 shows the time change in each detection signal of each of the fluorescence FL1 to FL3 output by each of the detectors 51 to 53. The detection signal is a signal representing the intensity of light of each of the fluorescence FL1 to FL3. Since the intensity of light of the fluorescence FL represents the leakage amount of the fluorescent tracer TR, the time change in the detection signal represents the time change in the leakage amount of the fluorescent tracer TR. The leakage amount of the fluorescent tracer TR is an example of the leakage state of each fluorescent tracer TR.

In addition, each of the detectors 51 to 53 can set, for example, the output interval of the detection signal to a short interval of about 1 ms at the maximum. The output interval is appropriately set according to the purpose.

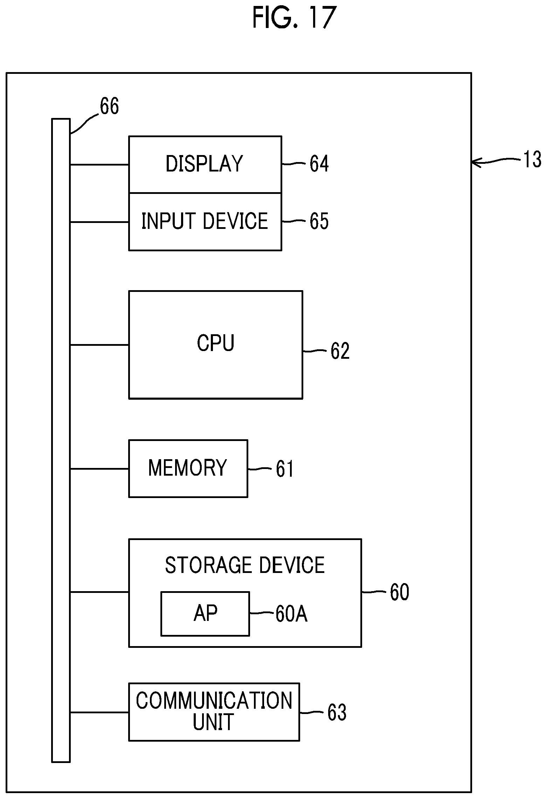

As shown in Fig. 17, the computer constituting the control device 13 includes a storage device 60, a memory 61, a central processing unit (CPU) 62, a communication unit 63, a display 64, and an input device 65. These are interconnected through a data bus 66.

The storage device 60 is a hard disk drive built in or connected through a cable, a network, or the like to the computer constituting the control device 13. Alternatively, the storage device 60 is a disk array in which a plurality of hard disk drives are connected in series. In addition, the storage device 60 may be arranged outside the computer and connected to the control device 13 through a network. The storage device 60 stores a control program such as an operating system, various application programs (AP) 60A, various data associated with these programs, and the like.

The memory 61 is a work memory for the CPU 62 to execute a process. The CPU 62 controls each part of the computer in an integrated manner by loading a program stored in the storage device 60 into the memory 61 and executing a process according to the program.

The communication unit 63 is a network interface that controls transmission of various information through a network such as a local area network (LAN), the Internet, or a wide area network (WAN) such as a public communication network. The display 64 displays various screens. Various screens are equipped with graphical user interface (GUI) operation functions. The computer constituting the control device 13 receives input of an operation instruction from the input device 65 through various screens. The input device 65 is a keyboard, a mouse, a touch panel, or the like.

The AP 60A includes an operation program for the measurement device 10. The operation program is a program for making the computer function as the control device 13 of the measurement device 10. In addition to the operation program, various data necessary for executing the operation program are stored in the storage device 60.

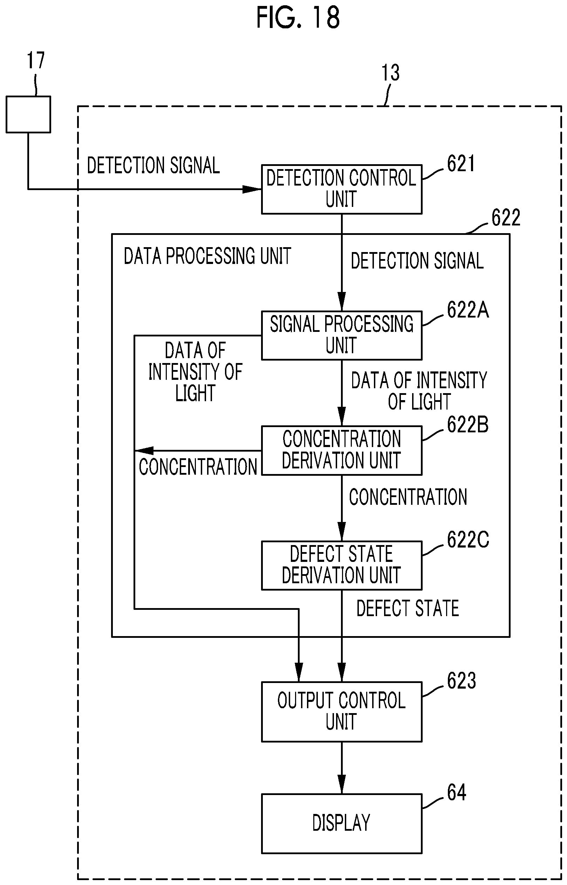

As shown in Fig. 18, in a case where the operation program is started, the CPU 62 of the computer constituting the control device 13 functions as a detection control unit 621, a data processing unit 622, and an output control unit 623 in cooperation with the memory 61 and the like. In addition, the data processing unit 622 functions as a signal processing unit 622A, a concentration derivation unit 622B, and a defect state derivation unit 622C.

The detection control unit 621 transmits a control signal to the detection unit 17 to thereby control the operation of each part of the detection unit 17, specifically, each of the excitation light source 56, the shutter unit 57, and the first detector 51 to the third detector 53. In addition, the detection control unit 621 acquires detection signals of the detectors 51 to 53 and outputs the acquired detection signals to the data processing unit 622. The detection control unit 621 functions as an acquisition unit that acquires a detection signal from the detection unit 17.

The data processing unit 622 carries out data processing on the detection signals output by each detection unit 17. In the data processing unit 622, the detection signal output from the detection control unit 621 is input to the signal processing unit 622A.

The signal processing unit 622A carries out a signal process on the detection signal. The signal process includes a correction process, a removal process for removing an overlapping portion, a conversion process, and the like. The correction process includes correction processes for the dark current of each of the light receiving sections 51A to 53A.

The overlapping portion removal process is a process of removing the portion where the wavelength ranges of a plurality of types of light overlap from the detection signals corresponding to the respective lights. In this example, the portion where the wavelength ranges of the plurality of types of fluorescence FL1 to FL3 overlap is removed from the detection signals corresponding to the fluorescence FL1 to FL3. As shown by hatching in Fig. 15, a part of the wavelength range of the fluorescence FL1 partially overlaps with the respective wavelength ranges of the fluorescence FL2 and FL3. Therefore, each of the detection signals corresponding to the fluorescence FL2 and FL3 includes an overlapping portion of the fluorescence FL1. In the removal process, the overlapping portion of the fluorescence FL1 is removed from the detection signals of the fluorescence FL2 and FL3.

For example, the correspondence relationship between the incidence ray amount at which the fluorescence FL1 is incident on the second detector 52 and the third detector 53 corresponding to the fluorescence FL2 and FL3 and the incidence ray amount at which the fluorescence FL1 is incident on the first detector 51 is measured in advance. Based on this correspondence relationship, the signal processing unit 622A calculates an overlapping portion according to the value of the detection signal output by the first detector 51. Then, the calculated overlapping portion is removed from the detection signals of the second detector 52 and the third detector 53.

In addition, the conversion process is a process of converting a detection signal representing a voltage value into data of intensity of light which is a detection signal representing an intensity of light.

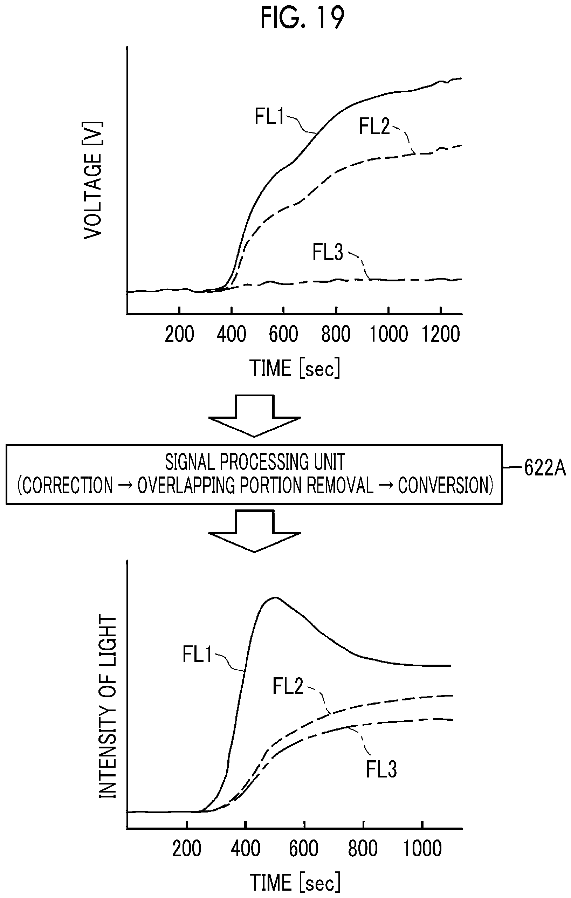

Fig. 19 shows an outline of the process of the signal processing unit 622A. As described above, the signal processing unit 622A executes each process of a correction process (correction) such as dark current correction, an overlapping portion removal process (overlapping portion removal), and a conversion process (conversion). By the conversion process, the time change in the voltage value is converted into the time change in the intensity of light. The signal processing unit 622A outputs the data of intensity of light to the concentration derivation unit 622B.

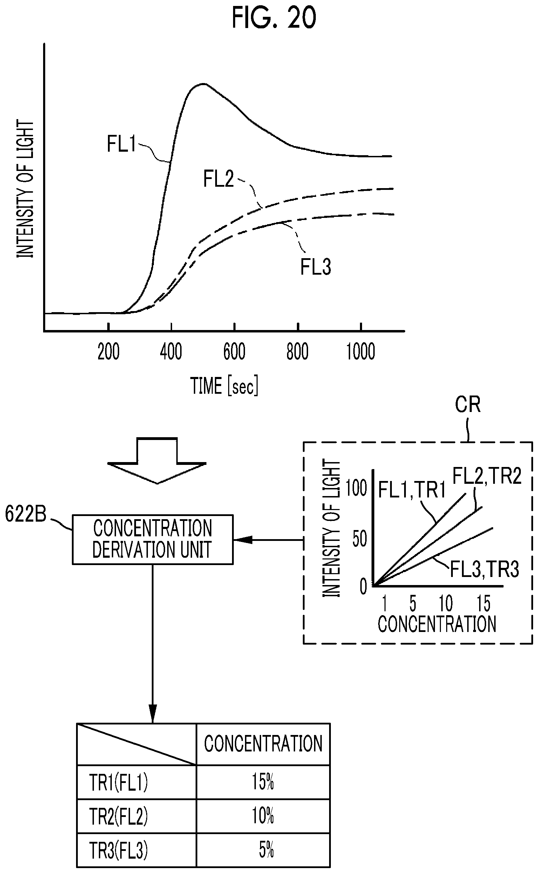

As shown in Fig. 20, the concentration derivation unit 622B acquires the data of intensity of light from the signal processing unit 622A. The concentration derivation unit 622B derives the respective concentrations of the plurality of types of fluorescent tracers TR1 to TR3 leaked out from the first flow channel 25 to the second flow channel 26, based on the data of intensity of light as the detection signal.

A calibration curve CR is used to derive the concentration. The calibration curve CR is stored in the storage device 60 as various data. The calibration curve CR shows the relationship between the respective concentrations of the fluorescent tracers TR1 to TR3 contained in the evaluation liquid 21 and the respective intensities of light of the fluorescence FL1 to FL3. The concentration is a concentration in a case where the respective concentrations of the fluorescent tracers TR1 to TR3 contained in the evaluation liquid 21 supplied to the first flow channel 25 are set to 100. The greater the leakage of each fluorescent tracer TR1 to TR3 from the first flow channel 25 to the second flow channel 26, the higher the concentration. Such a calibration curve CR is measured in advance, and the measured data is stored in the storage device 60.

Using the data of intensity of light and the calibration curve CR, the concentration derivation unit 622B derives a concentration such that the concentration of the fluorescent tracer TR1 is 15%, the concentration of the fluorescent tracer TR2 is 10%, and the concentration of the fluorescent tracer TR3 is 5%, as shown as an example in Fig. 20. The calibration curve CR may be stored as a table or as a function. The concentration derivation unit 622B outputs the derived concentration to the defect state derivation unit 622C.

In Fig. 18, the defect state derivation unit 622C derives the state of the defect occurring in the cell sheet 20 based on the concentration of each fluorescent tracer TR1 to TR3 derived by the concentration derivation unit 622B.

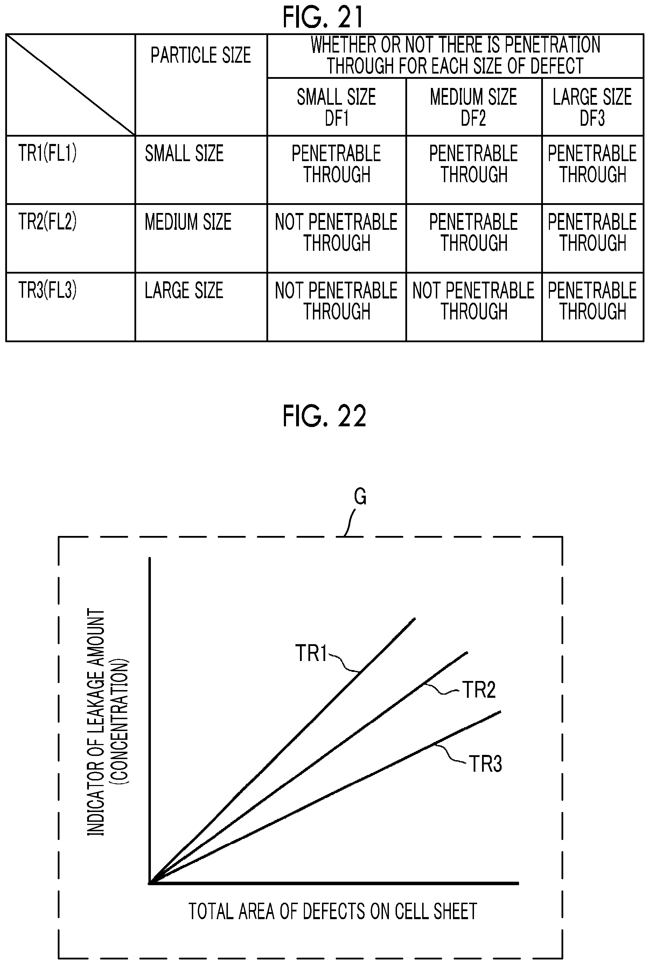

Fig. 21 shows the relationship between the sizes of the fluorescent tracers TR1 to TR3 and whether or not there is a penetration of the fluorescent tracers TR1 to TR3 for each size of the defect DF occurring in the cell sheet 20. The small size fluorescent tracer TR1 is capable of penetrating through all the defects DF of the small size defect DF1, the medium size defect DF2, and the large size defect DF3 shown in Fig. 9. The medium size fluorescent tracer TR2 is not capable of penetrating through the small size defect DF1, but is capable of penetrating through the medium size defect DF2 and the large size defect DF3. The large size fluorescent tracer TR3 is not capable of penetrating through the small size defect DF1 and the medium size defect DF2, and is capable of penetrating through only the large size defect DF3.

Thus, the small size fluorescent tracer TR1 is capable of penetrating through defects DF1 to DF3 of all sizes of small size, medium size and large size, and the medium size fluorescent tracer TR2 is capable of penetrating through medium size and large size defects DF2 and DF3. Therefore, it is not possible to grasp a state of defects occurring in the cell sheet 20, for example, an approximate proportion of a plurality of types of defects DF1 to DF3 having different sizes, only from the concentrations of the fluorescent tracers TR1 to TR3 of each size. That is, this is because the concentration of the fluorescent tracer TR1 does not depend only on the area of the small size defect DF1 since the small size fluorescent tracer TR1 penetrates not only through the small size defect DF1 but also the medium size defect DF2 and the large size defect DF3.

Therefore, the defect state derivation unit 622C derives the defect state by the following procedure. First, as shown in Fig. 22, a relationship G between the total area of the defect DF on the cell sheet 20 and the concentration which is an indicator of the leakage amount of each of the fluorescent tracers TR1 to TR3 is measured in advance. The relationship G is measured in a state where one type of each fluorescent tracer TR1 to TR3 is contained in the evaluation liquid 21. As shown in Fig. 9, the size of the defect DF can be various, but the total area of the defect DF in the relationship G is, for example, a total area of the defects DF having a size that allows each of the fluorescent tracers TR1 to TR3 to penetrate through.

That is, the relationship G shown in Fig. 22 represents how much each of the fluorescent tracers TR1 to TR3 leaks out into the second flow channel 26 in a case where how much the total area of the defects DF occurring in the cell sheet 20 is. The data representing this relationship G is stored in the storage device 60 in the form of a function or a table.

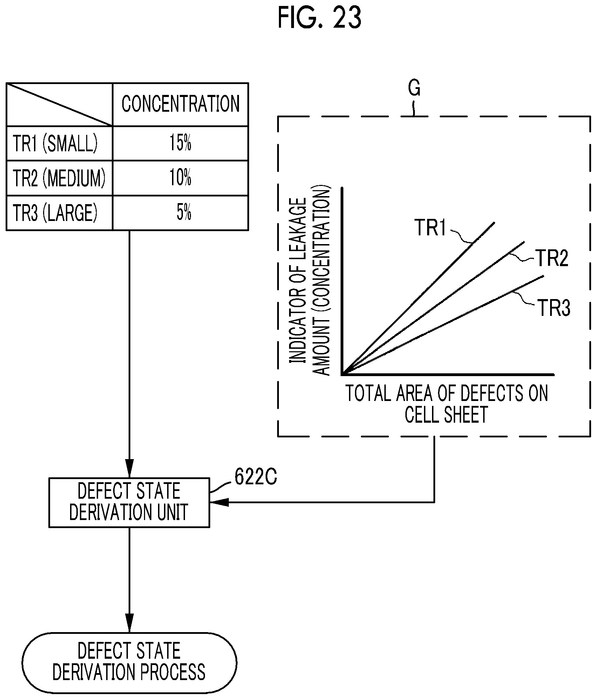

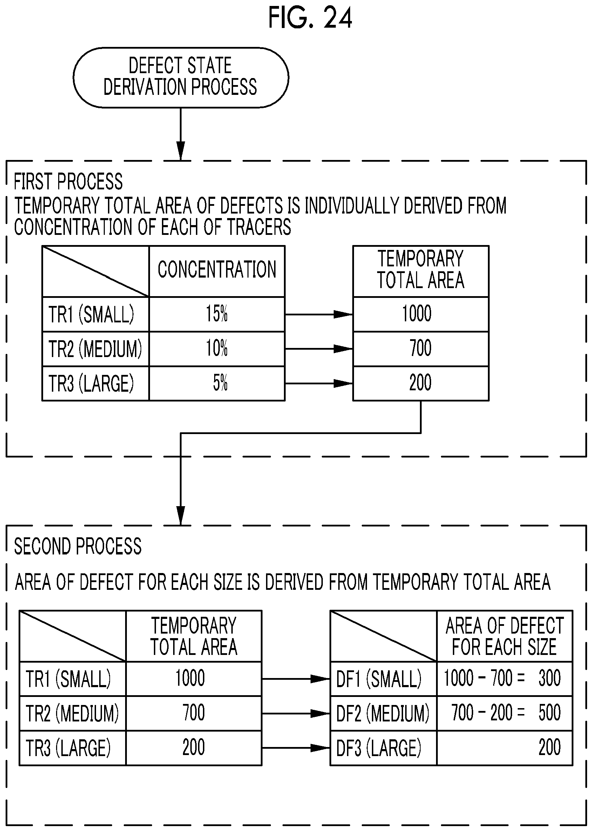

As shown in Fig. 23 and Fig. 24, the defect state derivation unit 622C executes the defect state derivation process using the measured value of the concentration derived by the concentration derivation unit 622B and the relationship G.

As shown in Fig. 24, in the defect state derivation process, the defect state derivation unit 622C first executes the first process. The first process is a process for individually deriving a temporary total area of the defect DF from the concentration of each of the fluorescent tracers TR1 to TR3. In the first process, the defect state derivation unit 622C obtains the total area corresponding to the concentration with reference to the relationship G, based on the respective concentrations of the fluorescent tracers TR1 to TR3. This total area is defined as the temporary total area.

As shown as an example in Fig. 24, in a case where the concentration of the small size fluorescent tracer TR1 is 15%, the temporary total area of the defects DF in the cell sheet 20 is 1000. In a case where the concentration of the medium size fluorescent tracer TR2 is 10%, the temporary total area of the defects DF is 700, and in a case where the concentration of the large size fluorescent tracer TR3 is 5%, the temporary total area of the defect DF is 200. In Fig. 24, the temporary total area is a standardized value as an example, and has no unit.

The defect state derivation unit 622C executes the second process after the first process. In the second process, the areas of the defects DF1 to DF3 for each size are derived from the temporary total area. The defect state derivation unit 622C obtains the area of the defects DF1 to DF3 having different sizes, in descending order of size. First, as shown in Fig. 21, the large size fluorescent tracer TR3 penetrates through only the large size defect DF3. Therefore, the temporary total area of 200 of the defects DF obtained based on the large size fluorescent tracer TR3 is considered to be the total area of only the large size defect DF3. Accordingly, the temporary total area of 200 obtained based on the large size fluorescent tracer TR3 is directly obtained as the area of the large size defect DF3.

Next, the defect state derivation unit 622C obtains the area of the medium size defect DF2. The medium size fluorescent tracer TR2 also penetrates through the large size defect DF3 in addition to the medium size defect DF2. Therefore, it is considered that the temporary total area of 700 of the defects DF obtained based on the medium size fluorescent tracer TR2 includes the area of the large size defect DF3. Accordingly, 200, which is the area of the large size defect DF3, is subtracted from the temporary total area of 700 obtained based on the medium size fluorescent tracer TR2. The defect state derivation unit 622C sets the thus obtained value of 500 as the area of the medium size defect DF2.

Finally, the defect state derivation unit 622C obtains the area of the small size defect DF1. The small size fluorescent tracer TR1 also penetrates through medium size and large size defects DF2 and DF3 in addition to the small size defect DF1. Therefore, it is considered that the temporary total area of 1000 of the defects DF obtained based on the small size fluorescent tracer TR1 includes the area of the medium size defect DF2 and the area of the large size defect DF3. Accordingly, 500, which is the area of the medium size defect DF2, and 200, which is the area of the large size defect DF3, are subtracted from the temporary total area of 1000 obtained based on the small size fluorescent tracer TR1. The defect state derivation unit 622C sets the thus obtained value of 300 as the area of the small size defect DF1.

In this way, the defect state derivation unit 622C derives, as a defect state, an approximate proportion of defects DF1 to DF3 having different sizes occurred in the cell sheet 20, based on the respective concentrations of the fluorescent tracers TR1 to TR3 having different sizes and the relationship G.

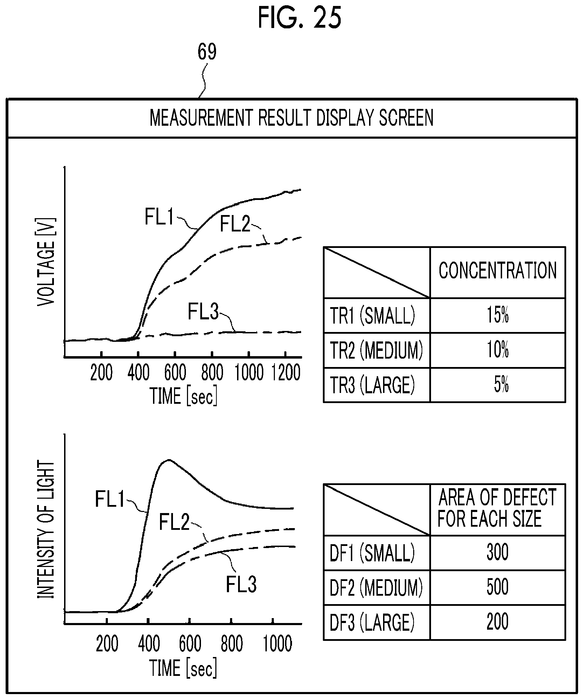

A measurement result display screen 69 shown in Fig. 25 is an example of a screen displayed on the display 64. The output control unit 623 shown in Fig. 18 generates such a measurement result display screen 69, and updates the contents of the measurement result display screen 69 in real time. As shown in Fig. 18, measurement data such as data of intensity of light processed by the signal processing unit 622A, the concentration derived by the concentration derivation unit 622B, and the defect state derived by the defect state derivation unit 622C are input to the output control unit 623. Such measurement data represent the leakage state of the fluorescent tracers TR1 to TR3, and serve as an indicator for evaluating the barrier properties of the cell sheet 20.

The output control unit 623 displays such measurement data as the measurement results on the measurement result display screen 69. The measurement data such as data of intensity of light, concentration, and defect state are updated based on detection signals acquired in real time. The output control unit 623 displays the measurement data in real time. As a result, the leakage state of the fluorescent tracers TR1 to TR3 is output to the display 64 in real time. The output control unit 623 functions as a processing unit that executes an output control process for outputting the leakage state.

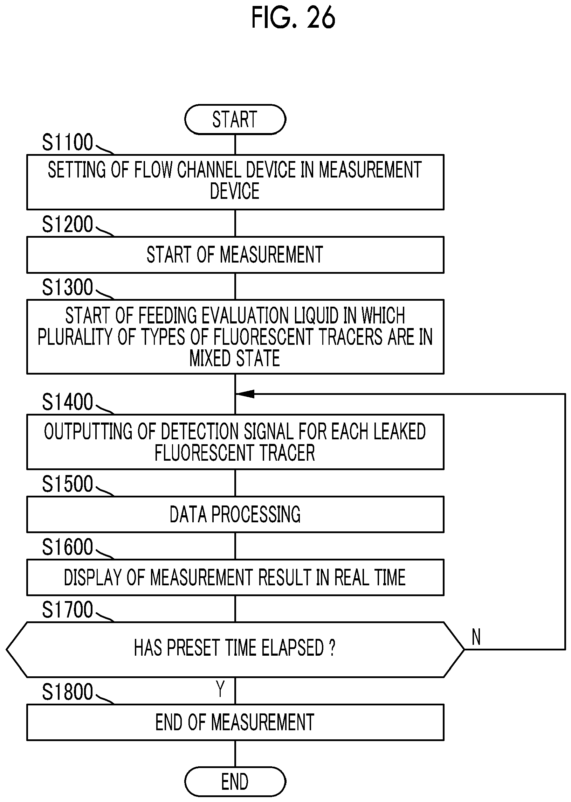

Hereinafter, the operation of the above-mentioned configuration will be described with reference to the flowchart shown in Fig. 26. In this example, the user of the measurement device 10 is the manufacturer of the flow channel device 11. The user uses the measurement device 10 in a case of evaluating the barrier properties of the cell sheet 20 of the produced flow channel device 11.

First, in step S1100, the flow channel device 11 is set in the measurement device 10. Specifically, the flow channel device 11 is mounted on the mounting unit 16. Then, as shown in Fig. 1, the supply tube 22A and the discharge tube 22B are attached to the flow channel device 11. Further, the evaluation liquid 21 in which three types of fluorescent tracers TR1 to TR3 are in a mixed state is adjusted in advance, and the adjusted evaluation liquid 21 is set in the tank of the pump 22C.

In step S1200, the measurement device 10 starts the measurement in a case where the measurement start instruction is input by an operation instruction such as a measurement start button. Specifically, the detection control unit 621 starts the operation of individual units such as the excitation light source 56 and the first detector 51 to the third detector 53 of the detection unit 17. As a result, the output of the detection signal is started from each of the first detector 51 to the third detector 53.

In step S1300, in a case where the operation of the pump 22C is started, the evaluation liquid 21 in which three types of fluorescent tracers TR1 to TR3 are in a mixed state as a plurality of types of tracers is supplied to the flow channel device 11. As a result, the feeding of the evaluation liquid 21 to the first flow channel 25 is started.

In step S1400, the first detector 51 to the third detector 53 of the detection unit 17 each output detection signals. In a case where the cell sheet 20 is free of defect DF, there is no leakage of the fluorescent tracer TR into the second flow channel 26. In this case, the first detector 51 to the third detector 53 output a detection signal indicating a baseline.

On the other hand, in a case where defect DF occurred in the cell sheet 20, at least one of three types of fluorescent tracers TR1 to TR3 present in a mixed state in the evaluation liquid 21 penetrates through the defect DF and leaks out from the first flow channel 25 to the second flow channel 26. From the detection unit 17, the detection position 46 of the second flow channel 26 is irradiated with the excitation light LB. In a case where the fluorescent tracers TR1 to TR3 leak out into the second flow channel 26, the fluorescent tracers TR1 to TR3 are irradiated with the excitation light LB to thereby emit each fluorescence FL1 to FL3 of the fluorescent tracers TR1 to TR3. The fluorescence FL1 to FL3 are optically separated by the dichroic mirrors DM1 to DM3 in the first lens barrel unit 54 of the detection unit 17, and are incident on each of the first detector 51 to the third detector 53.

The first detector 51 to the third detector 53 output a detection signal for each leaked fluorescent tracer TR according to the intensity of light of each of the fluorescence FL1 to FL3 corresponding to each of three types of fluorescent tracers TR1 to TR3. That is, the intensity of light of each of the fluorescence FL1 to FL3 represents the leakage state of each of the fluorescent tracers TR1 to TR3. That is, the detection unit 17 optically distinguishes and detects the leakage state of each fluorescent tracer TR1 to TR3 by distinguishing and detecting the intensity of light of each fluorescence FL1 to FL3. The detection control unit 621 acquires a detection signal from the detection unit 17 and outputs the acquired detection signal to the data processing unit 622.

In step S1500, the data processing unit 622 carries out data processing on the detection signal. As shown in Fig. 19, the signal processing unit 622A executes correction process, overlap removal process, conversion process, and the like on the detection signal. The concentration derivation unit 622B executes the concentration derivation process shown in Fig. 20 on the signal-processed data of intensity of light to derive the concentration. The defect state derivation unit 622C executes the defect state derivation process shown in Fig. 23 and Fig. 24 based on the concentration derived by the concentration derivation unit 622B to derive the defect state.

In step S1600, the output control unit 623 generates the measurement result display screen 69 using the measurement data processed by the data processing unit 622 as the measurement result, and displays the measurement result on the display 64 in real time. Based on the measurement result of the measurement result display screen 69, the user can check the leakage state including the presence or absence of leakage of each fluorescent tracer TR1 to TR3, the leakage amount and the time change in the leakage amount, and further the defect state of the cell sheet 20. Since such a measurement result is an indicator for evaluating the barrier properties of the cell sheet 20, the user evaluates the barrier properties of the cell sheet 20 based on the measurement result.

In step S1700, in a case where the preset time has not elapsed (N in S1700), the measurement process of steps S1400 to S1600 is continued. In step S1700, in a case where the preset time has elapsed (Y in S1700), the process proceeds to step S1800. In step S1800, the measurement is completed by stopping the feeding of the evaluation liquid and the operation of the detection unit 17.

The measurement device 10 according to the technique of the present disclosure has the detection unit 17 that optically distinguishes and detects the leakage state of each of a plurality of fluorescent tracers TR1 to TR3 leaking out from the first flow channel 25 to the second flow channel 26 through the defect DF occurring in the cell sheet 20, in a case where the evaluation liquid 21 in which the three types of fluorescent tracers TR1 to TR3 are in a mixed state as a plurality of types of tracers having different sizes is fed to the first flow channel 25. Therefore, it is possible to detect a plurality of types of defects DF having different sizes occurring in the cell sheet 20 without taking time and effort as compared with the related art.

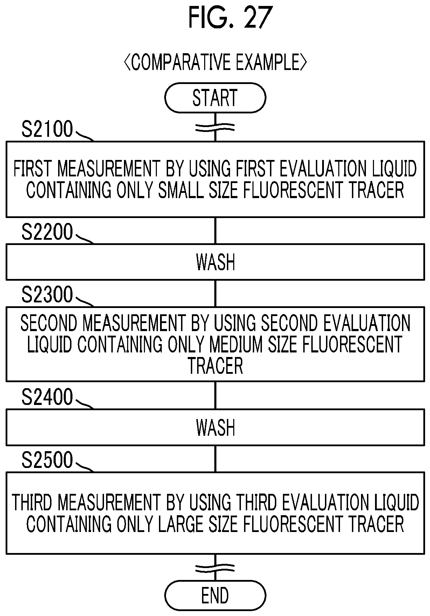

The effects of the measurement device 10 according to the technique of the present disclosure will be described in more detail with reference to the comparative example shown in Fig. 27. The comparative example shown in Fig. 27 is the same as that of the measurement device 10 in that three types of fluorescent tracers TR1 to TR3 are used. The difference therebetween is that the measurement is carried out using one type of each fluorescent tracer TR1 to TR3 without using the evaluation liquid 21 in which these fluorescent tracers TR1 to TR3 are in a mixed state.

More specifically, in the comparative example, three types of evaluation liquids, a first evaluation liquid containing only the small size fluorescent tracer TR1, a second evaluation liquid containing only the medium size fluorescent tracer TR2, and a third evaluation liquid containing only the large size fluorescent tracer TR3, are used. Then, in the comparative example, the measurement is carried out in three steps while exchanging these first evaluation liquid, second evaluation liquid and third evaluation liquid in order.

That is, first, in step S2100, a first measurement is carried out using the first evaluation liquid containing only the small size fluorescent tracer TR1. The leakage state of the small size fluorescent tracer TR1 is detected by carrying out the first measurement.

Before carrying out the next measurement, in step S2200, a buffer is flowed through the first flow channel 25 to wash the first flow channel 25.

In step S2300, a second measurement is carried out using the second evaluation liquid containing only the medium size fluorescent tracer TR2. The leakage state of the medium size fluorescence FL2 is detected by carrying out the second measurement.

Before carrying out the next measurement, in step S2400, a buffer is flowed through the first flow channel 25 to wash the first flow channel 25.

Finally, in step S2300, a third measurement is carried out using the third evaluation liquid containing only the large size fluorescent tracer TR3. The leakage state of the large size fluorescence FL3 is detected by carrying out the third measurement.

The leakage state of each fluorescent tracer TR1 to TR3 can also be detected by a method of using the three types of evaluation liquids while exchanging each thereof in order as in the comparative example.

However, in the comparative example, it takes more time and effort as compared with the measurement device 10 and the measurement method according to the technique of the present disclosure, since it is necessary to carry out the measurement three times while exchanging the three types of evaluation liquids in order. In addition, in the comparative example, it is difficult to detect the leakage state of each fluorescent tracer TR1 to TR3 under the same conditions, since the measurement is carried out three times while exchanging the three types of evaluation liquids in order. This is because the cell sheet 20 is not a little damaged every time the evaluation liquid is fed. This is because the defect state of the cell sheet 20 changes in each measurement due to this damage.

Further, in a case where different types of evaluation liquids are exchanged in order as in the comparative example, it is necessary to wash the first flow channel 25 each time the evaluation liquid is exchanged. The cell sheet 20 is also damaged by washing, since the cell sheet 20 and the buffer come into contact with each other during washing. In addition, the time and effort required for washing will increase.

The measurement device 10 according to the technique of the present disclosure uses an evaluation liquid in which a plurality of types of tracers are in a mixed state to detect the leakage state of each tracer in one measurement. Therefore, the effects capable of not only reducing the time and effort, but also detecting the leakage state of each tracer under the same conditions as compared with the comparative example can be obtained.

In addition, fluorescent tracers TR1 to TR3 that emit fluorescence having different wavelength ranges are used as a plurality of types of tracers. It is easy to select an appropriate tracer since the fluorescent tracer TR has a wide variety of sizes and fluorescent colors.

In addition, the detection unit 17 for detecting fluorescence has the excitation light source 56, the dichroic mirrors DM1 to DM3 as optical filters for separating a plurality of types of fluorescence FL1 to FL3, and the light receiving section 51A to the light receiving section 53A that receives the fluorescence FL1 to FL3 separated by each dichroic mirror DM1 to DM3. Such a configuration for detecting fluorescence is common and therefore the detection unit 17 can be easily produced.

In addition, since the measurement device 10 uses a laser light source as the excitation light source 56, it is possible to detect the fluorescence FL1 to FL3 having a strong intensity of light. Therefore, the S/N ratio of the detection signal is improved. In addition, photomultipliers are used as the first detector 51 to the third detector 53. Therefore, the S/N ratio of the detection signal is improved as compared with the use of a fluorescence microscope.

In addition, the measurement device 10 has one excitation light source 56, and is provided with three light receiving sections, the light receiving section 51A to the light receiving section 53A, for each of the three types of fluorescence FL1 to FL3. By including one excitation light source 56, it is possible to achieve simplified configuration, downsizing, and reduced production costs as compared with the case where a plurality of excitation light sources 56 are provided. In addition, since the three light receiving sections are provided, for example, it is easy to adjust according to the wavelength range of each fluorescence FL1 to FL3.

In addition, the measurement device 10 according to the technique of the present disclosure irradiates the detection position 46, in the flow channel device 11 mounted on the mounting unit 16, with the excitation light LB. The detection position 46 is on the downstream side in the flow direction of the fluorescent tracers TR1 to TR3, which are examples of tracers leaking out from the communicating portion corresponding to the slit 34A, and is an example of the position where the light is incident only on the second flow channel 26 of the first flow channel 25 and the second flow channel 26. Since the excitation light LB is not incident on the first flow channel 25, only the tracer leaking out to the second flow channel 26 can be reliably detected.

The leakage state detected by the measurement device 10 according to the technique of the present disclosure includes a time change in the leakage amount of the fluorescent tracers TR1 to TR3, which are examples of tracers. Therefore, it is possible to observe the time change in the defect state of the cell sheet 20.

In addition, the measurement device 10 according to the technique of the present disclosure includes the data processing unit 622 that carries out data processing on the detection signal output by the detection unit 17. As a result, the detection signal can be processed into a form suitable for observation, such as converting the detection signal representing a voltage value into data of intensity of light. In addition, various measurement results such as concentration and defect state can be obtained by the data processing unit 622.

In addition, in the measurement device 10, the signal processing unit 622A executes a correction process for the detection signal and a removal process for removing the overlapping portion of wavelength ranges of the fluorescence FL1 to FL3 as a plurality of types of light from the detection signal, whereby a more accurate detection signal can be obtained.

For example, the graph shown in the upper part of Fig. 19 is a graph showing the time change in the voltage value before the overlapping portion removal process is executed. In addition, the graph shown in the lower part of Fig. 19 is a graph showing the time change in the intensity of light after the overlapping portion removal process is executed. In the upper graph, all three types of fluorescence FL, fluorescence FL1 to fluorescence FL3, appear to increase monotonically.

However, in the graph shown in the lower part of Fig. 19, the intensity of light of the fluorescence FL1 does not increase monotonically. Specifically, the intensity of light of fluorescence FL1 increases at first, but decreases after reaching a peak. By carrying out the overlapping portion removal process in this way, it is possible to grasp the accurate time change in each detection signal indicating the leakage state of the fluorescent tracer TR.

In addition, in the measurement device 10, the concentration derivation unit 622B derives the respective concentrations of the fluorescent tracers TR1 to TR3 which are examples of a plurality of types of tracers. Therefore, it is easier to intuitively grasp the leakage state of the fluorescent tracer TR by indicating the leakage state in terms of concentration as compared with the case where the leakage state of the fluorescent tracer TR is displayed by the voltage value.

In the measurement device 10, the defect state derivation unit 622C derives the defect state which is a state of the defect DF occurring in the cell sheet 20, based on the respective concentrations of the fluorescent tracers TR1 to TR3 which are examples of a plurality of types of tracers. The defect state includes, for example, an approximate proportion of each of a plurality of types of defects DF1 to DF3 having different sizes. By deriving such a defect state, the barrier properties of the cell sheet 20 can be evaluated in detail as compared with the case of simply detecting the presence or absence of the defect DF.

In the measurement device 10, the output control unit 623 displays the leakage state of each of the fluorescent tracers TR1 to TR3, which are examples of tracers, detected by the detection unit 17 on the display 64 in real time. Thereby, the time change in the leakage state of the fluorescent tracer TR and the time change in the defect state of the cell sheet 20 can be observed in real time.

In addition, in the flow channel device 11, the permeable membrane 36 that functions as a support for the cell sheet 20 has pores having a diameter of 1 µm or more. In the related art JP2018-522586A, a gel is used as the support for the cell sheet 20. The gel does not easily allow for a large size tracer of 1 µm or more to penetrate therethrough. Therefore, using the permeable membrane 36 makes it possible to detect a large size defect DF of 1 µm or more occurring in the cell sheet 20.

As the support for the cell sheet 20, a gel may be used instead of the permeable membrane 36. In a case where a gel is used as the support for the cell sheet 20, the size of the tracer may be selected according to the properties of the gel. However, it is preferable to use the permeable membrane 36 since the permeable membrane 36 has the above-mentioned advantages.

In addition, the size (particle size) of each of the fluorescent tracers TR1 to TR3, which are examples of a plurality of types of tracers, is within a range of 1 nm or more and 100 µm or less. This makes it possible to detect defects DF of various sizes in the cell sheet 20.

The fluorescent tracers TR1 to TR3, which are examples of a plurality of types of tracers, include tracers having a size (particle size) on the order of 1 nm to several hundreds of nm and tracers having a size (particle size) on the order of 1 µm to several tens of µm. In a case where it is desired to detect the defects DF having different sizes, there is little advantage in using a plurality of types of tracers that do not have a large difference in size. By using tracers having a larger difference in size (particle size) as the order is different, it is possible to efficiently detect the defects DF having different sizes.

The measurement device 10 includes the supply mechanism 22 for supplying the evaluation liquid 21 to the first flow channel 25. Therefore, the evaluation liquid 21 can be easily fed as compared with the case where the supply mechanism is separately prepared. The measurement device 10 may not be provided with the supply mechanism 22. It is also possible to substitute the supply mechanism 22 with a syringe, a pipette, or the like.

### (Modification Example 1: cell sheets are formed on both surfaces of permeable membrane)

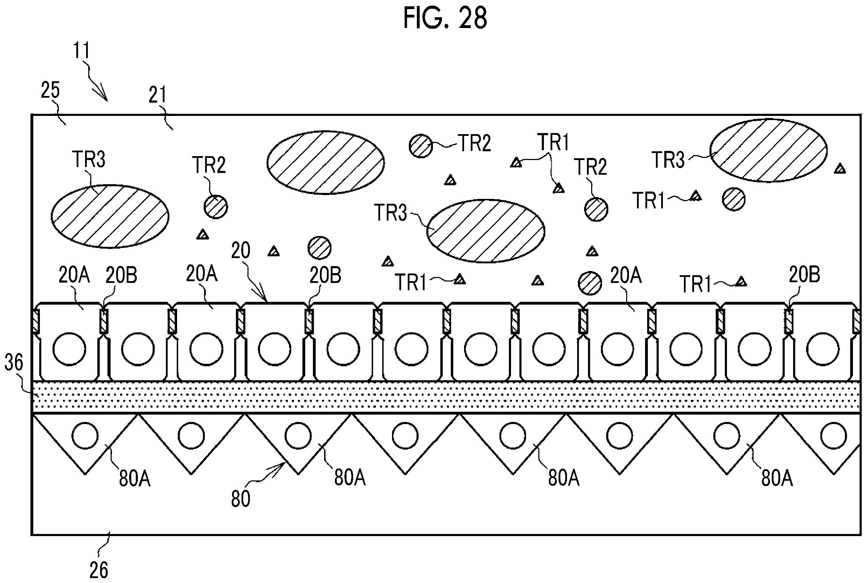

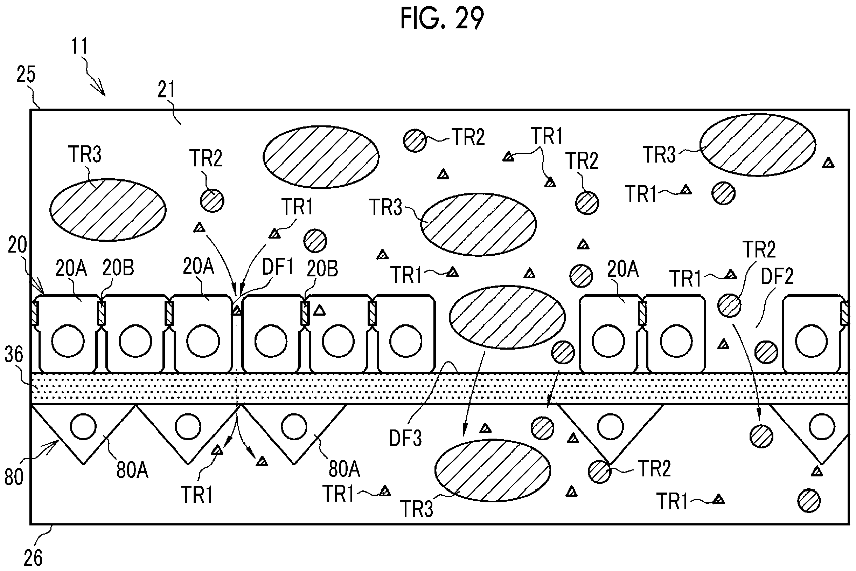

As shown in Fig. 8 and Fig. 9, in the flow channel device 11 of the foregoing example, an example has been described in which the cell sheet 20 is formed only on one surface of the permeable membrane 36, that is, the surface on the first flow channel 25 side. As shown in Fig. 28 and Fig. 29, a cell sheet 80 may also be formed on the other surface of the permeable membrane 36, that is, the surface on the second flow channel 26 side. That is, the examples shown in Fig. 28 and Fig. 29 are examples in which the cell sheets are formed on both surfaces of the permeable membrane 36.

The cell sheet 80 is, for example, an array of cells 80A, and the cells 80A are smooth muscle cells (SMCs). The tissues that make up the inner wall of blood vessels include endothelial cells formed on one surface of the basement membrane and smooth muscle cells formed on the other surface of the basement membrane. Therefore, in a case of simulating a blood vessel using the flow channel device 11, it is possible to get closer to the actual composition of the blood vessel tissue in such a manner that the permeable membrane 36 is regarded as a basement membrane, the cell sheet 20 is formed on one surface of the permeable membrane 36, and the cell sheet 80 is formed on the other surface of the permeable membrane 36, as shown in Fig. 28 and Fig. 29.

The cell 80A, which is a smooth muscle cell, has no tight junction, unlike the cell 20A, which is an endothelial cell. Therefore, as shown in Fig. 29, even in a case where a plurality of cells 80A are arranged in the cell sheet 80, the small size fluorescent tracer TR1 can penetrate through the gaps of the cells 80A. In addition, the cell sheet 80 is easier to peel off than the cell sheet 20.

### (Modification Example 2: rotation filter)

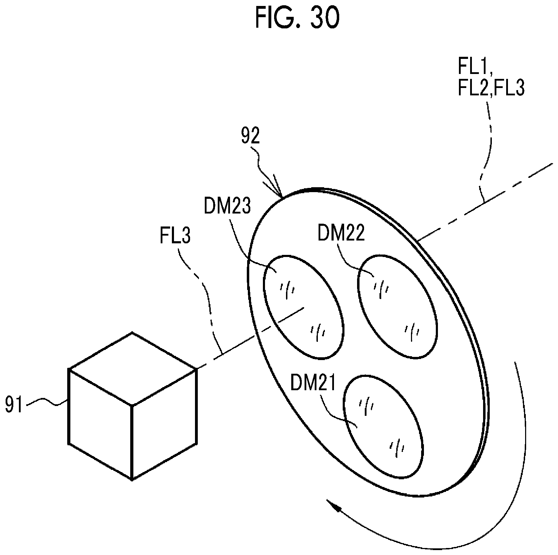

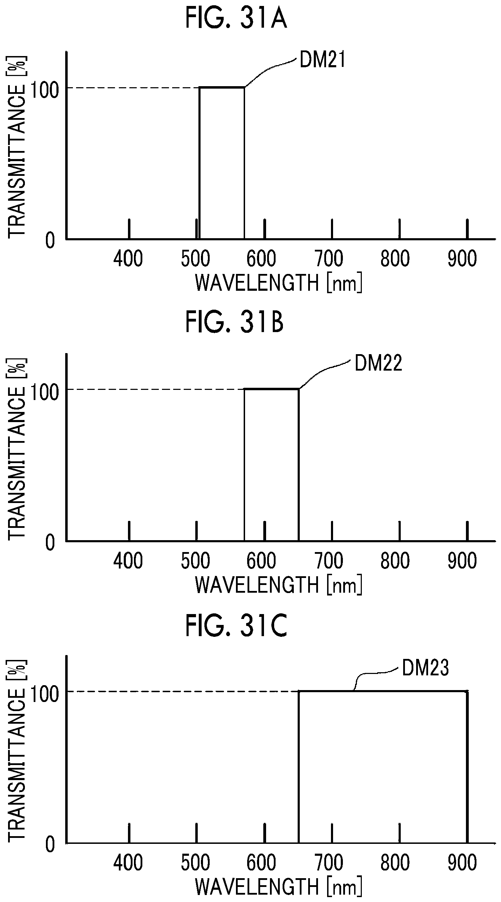

In the foregoing example, an example has been described in which a plurality of first detectors 51 to third detectors 53 are provided corresponding to the fluorescence FL1 to FL3 of the fluorescent tracers TR1 to TR3. However, as shown in Fig. 30 and Fig. 31, using a rotation filter 92 makes it possible to detect three types of fluorescence FL1 to FL3 with one detector 91.

The rotation filter 92 is provided with a plurality of types of dichroic mirrors DM21 to DM23 as optical filters having different transmission properties. The rotation filter 92 is rotated by driving a motor (not shown), for example. By this rotation, the dichroic mirrors DM21 to DM23 are selectively inserted into the optical paths of the fluorescence FL1 to FL3.

In Fig. 31, as shown in Fig. 31A, the dichroic mirror DM21 is a bandpass filter having transmission properties that transmit only the wavelength range of the fluorescence FL1. As shown in Fig. 31B, the dichroic mirror DM22 is a bandpass filter having transmission properties that transmit only the wavelength range of the fluorescence FL2. As shown in Fig. 31C, the dichroic mirror DM23 is a bandpass filter having transmission properties that transmit only the wavelength range of the fluorescence FL3.

The combination of the wavelength range of the fluorescence and the size of the fluorescent tracer shown in the foregoing example is an example, and such a combination is appropriately selected. For example, the size of the fluorescent tracer is selected according to the characteristic features of the defect DF, such as the size of the defect DF occurring in the cell sheet. In addition, the wavelength range of the fluorescence is also appropriately selected according to the characteristic features of the optical filter of the detection unit 17, the light receiving section of the detector, and the like.

Although an example of using three types of fluorescent tracers TR as a plurality of types of tracers has been described, the number of tracers is not limited to three types, and may be two types or four or more types.

In addition, in the foregoing example, an example of using a fluorescent tracer as an example of the tracer and using the fluorescence emitted by the fluorescent tracer to optically distinguish the leakage state of each tracer has been described, but Raman scattered light may be used instead of fluorescence. In a case where a substance is irradiated with light, the light interacts with the substance to emit light called Raman scattered light, which has a wavelength different from that of the incidence ray. The Raman scattered light is light that has a spectrum peculiar to a substance. In a case where the Raman scattered light is used, a plurality of types of tracers of different sizes that emit Raman scattered light having different spectra are used as the plurality of types of tracers. Then, the leakage state of each tracer is optically distinguished based on the Raman scattered light emitted by each of the plurality of types of tracers.

It should be noted that such Raman scattered light is very weak, and the device configuration is complicated as compared with the configuration of the detection unit 17 that detects fluorescence. Therefore, it is preferable that the measurement device 10 utilizes fluorescence.

In each of the foregoing embodiments, the following various processors can be used as a hardware-like structure of the processing unit that executes various processes such as the detection control unit 621 and the data processing unit 622. The various processors include, for example, a central processing unit (CPU), which is a general-purpose processor that executes software and functions as various processing units; as well as a programmable logic device (PLD), which is a processor whose circuit configuration can be changed after production, such as a field programmable gate array (FPGA), and/or a dedicated electric circuit, which is a processor having a circuit configuration specially designed for executing a specific process, such as an application specific integrated circuit (ASIC)

One processing unit may be configured of one of these various processors, or may be configured of a combination of two or more processors of the same type or different types (for example, a combination of a plurality of FPGAs and/or a combination of a CPU and an FPGA). In addition, a plurality of processing units may be configured into a single processor.

As an example in which a plurality of processing units are configured into a single processor, there is a form in which a single processor is configured by a combination of one or more CPUs and software, and this processor functions as a plurality of processing units. Secondly, as typified by a system on chip (SoC) or the like, there is a form in which a processor that realizes the functions of the entire system including a plurality of processing units with a single integrated circuit (IC) chip is used. As described above, the various processing units are configured by using one or more of the above-mentioned various processors as the hardware-like structure.

Further, as the hardware-like structure of these various processors, more specifically, a circuitry in which circuit elements such as semiconductor elements are combined can be used.

In addition, the technique of the present disclosure extends to an operation program for a measurement device; as well as a computer-readable storage medium that non-temporarily stores the operation program for a measurement device (such as universal serial bus (USB) memory or digital versatile disc (DVD)-read only memory (ROM)).

In the present specification, "A and/or B" is synonymous with "at least one of A or B." That is, "A and/or B" means that it may be only A, only B, or a combination of A and B. In addition, in the present specification, the same concept as "A and/or B" is applied in a case where three or more matters are connected and expressed by "and/or".

The contents described and illustrated hereinbefore are detailed explanations of the parts according to the technique of the present disclosure, and are merely examples of the technique of the present disclosure. For example, the foregoing description of the configuration, function, action, and effect is a description of an example of the configuration, function, action, and effect of the parts according to the technique of the present disclosure. Therefore, it goes without saying that unnecessary parts may be deleted, new elements may be added, or replacements may be made to the described contents and illustrated contents shown hereinbefore within a range that does not deviate from the gist of the technique of the present disclosure. In addition, in order to avoid confusion and facilitate understanding of the parts according to the technique of the present disclosure, explanations regarding common general technical knowledge and the like, which do not require any particular explanation in order to enable the implementation of the technique of the present disclosure, are omitted in the described contents and illustrated contents shown hereinbefore.

## Claims

1. A measurement device comprising:
a mounting unit in which a flow channel device is attachably and detachably mounted, the flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion; and
a detection unit that optically distinguishes and detects a leakage state of each of a plurality of types of tracers having different sizes and leaking out from the first flow channel into the second flow channel through a defect occurring in the cell sheet in a case where an evaluation liquid is fed into the first flow channel,
the evaluation liquid being used for evaluation of barrier properties of the cell sheet and containing the plurality of types of tracers in a mixed state.

2. The measurement device according to claim 1,
wherein each of the plurality of types of tracers is a fluorescent tracer which emits fluorescence having a different wavelength range, and
the detection unit detects a plurality of types of fluorescence emitted by each of the plurality of types of tracers.

3. The measurement device according to claim 2,
wherein the detection unit includes an excitation light source for irradiating each of the plurality of types of tracers with excitation light that excites the fluorescence,
an optical filter for separating the plurality of types of fluorescence, and
a light receiving section for receiving the fluorescence separated by the optical filter.

4. The measurement device according to claim 3,
wherein the number of the excitation light source is one, and
the light receiving section is provided for each of the plurality of types of fluorescence.

5. The measurement device according to claim 4,
wherein the excitation light source is on a downstream side in a flow direction of the tracer leaking out from the communicating portion in the flow channel device mounted on the mounting unit, and
irradiates a position where light is incident only on the second flow channel of the first flow channel and the second flow channel with excitation light.

6. The measurement device according to any one of claims 1 to 5,
wherein the leakage state includes a time change in a leakage amount.

7. The measurement device according to any one of claims 1 to 6, further comprising a data processing unit that carries out data processing on detection signals output by the detection unit.

8. The measurement device according to claim 7,
wherein the data processing unit has a signal processing unit that executes at least one of a correction process for correcting a dark current of the detection unit, a removal process for removing an overlapping portion of a wavelength range of the plurality of types of light from the detection signal corresponding to each light, or a conversion process for converting the detection signal output as a voltage signal into an intensity of light.

9. The measurement device according to claim 7 or 8,
wherein the data processing unit has a concentration derivation unit for deriving a concentration of each of the plurality of types of tracers leaked out from the first flow channel to the second flow channel based on the detection signal output by the detection unit.

10. The measurement device according to claim 9,
wherein the data processing unit has a defect state derivation unit for deriving a defect state occurring in the cell sheet based on the concentration of each of the plurality of types of tracers.

11. The measurement device according to any one of claims 1 to 10, further comprising an output control unit for outputting the leakage state detected by the detection unit to a display in real time.

12. The measurement device according to any one of claims 1 to 11,
wherein the cell sheet is cultured on a support, and
the support is a permeable membrane having pores having a diameter of 1 µm or more.

13. The measurement device according to claim 12,
wherein a particle size of each of the plurality of types of tracers is within a range of 1 nm or more and 100 µm or less.

14. The measurement device according to claim 13,
wherein the plurality of types of tracers include a tracer having a particle size on the order of 1 nm to several hundreds of nm and a tracer having a particle size on the order of 1 µm to several tens of µm.

15. The measurement device according to any one of claims 1 to 14, further comprising a supply mechanism for supplying the evaluation liquid to the first flow channel.

16. An operation program for a measurement device for evaluating barrier properties of a cell sheet using a flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion,
the operation program causing a computer to function as:
an acquisition unit for acquiring a detection signal from a detection unit that optically distinguishes and detects a leakage state of each of a plurality of types of tracers having different sizes and leaking out from the first flow channel into the second flow channel through a defect occurring in the cell sheet in a case where an evaluation liquid is fed into the first flow channel, the evaluation liquid being used for evaluation of the barrier properties of the cell sheet and containing the plurality of types of tracers in a mixed state; and
a processing unit for executing an output control process that outputs the leakage state based on the acquired detection signal.

17. A measurement method using a flow channel device including a first flow channel, a second flow channel having a communicating portion that communicates with at least a part of the first flow channel, and a cell sheet in which a plurality of cultured cells are arranged in such a manner that the cells are bound to each other and which partitions between the first flow channel and the second flow channel at the communicating portion,
the measurement method comprising:
a liquid feeding step of feeding an evaluation liquid to the first flow channel, the evaluation liquid being used for evaluating barrier properties of the cell sheet and containing a plurality of types of tracers having different sizes in a mixed state; and
a detection step of optically distinguishing and detecting a leakage state of each of the plurality of types of tracers leaking out from the first flow channel into the second flow channel through a defect occurring in the cell sheet.
